# EUROPEAN PATENT SPECIFICATION

(11) **EP 3 481 495 B1**
(45) Date of publication and mention of the grant of the patent: **18.11.2026**
(21) Application number: 17823765.7
(22) Date of filing: 05.07.2017
(51) Int. Cl.: A61N 1/32, A61N 1/05, A61N 1/06, A61N 1/40, A61N 1/04, A61H 19/00, A61F 7/00, A61N 2/00, A61N 1/36, A61B 18/00, A61B 18/14, A61B 18/12

(54) **AN ESTHETIC APPARATUS USEFUL FOR INCREASING SKIN REJUVENATION**
ZUR ERHÖHUNG DER HAUTVERJÜNGUNG NÜTZLICHE, ÄSTHETISCHE VORRICHTUNG
APPAREIL À USAGE ESTHÉTIQUE UTILE POUR ACCROÎTRE LA RÉGÉNÉRATION DE LA PEAU

(30) Priority: 07.07.2016 US 201615204158; 07.07.2016 US 201662359236 P; 13.07.2016 IT 201600073116
(43) Date of publication of application: 15.05.2019
(73) Proprietor: Venus Concept Ltd, 2165254 Karmiel (IL)
(72) Inventor: VAYNBERG, Boris, 30900 Zichron Yakov (IL)
(74) Representative: Dehns
(86) International application number: PCT/IL2017/050748
(87) International publication number: WO 2018/008023

(56) References cited:
- EP-A1- 2 564 894
- WO-A2-2010/007614
- WO-A2-2011/034986
- US-A1- 2012 022 504
- US-A1- 2013 317 282
- US-B2- 8 961 511

## Description

### FIELD OF THE INVENTION

This invention generally relates to an esthetic device used to improve viability and rejuvenation of mucosal tissue.

### BACKGROUND OF THE INVENTION

As a woman ages, mucosal tissue, such as the tissue in the vagina, ages in a manner similar to the aging of external skin tissues, experiencing a decrease in collagen production and reduced elasticity. This decrease in elasticity can lead to the vagina not responding properly to hormonal changes. This can lead to painful intercourse, if the tissues do not expand properly, or a decrease in sensation during intercourse, if the tissues do not contract properly.

A few main approaches to tightening of the tissues are common practice today. The surgical approach carries disadvantages related to the anesthesia, the surgical complications, and the healing process, which may cause scars. The chemical peel approach usually involves injury to the outermost layer of the skin - the epidermis - which may cause discoloration. Since collagen fibers are found in the dermis - the subcutaneous layer of the skin, and since heat was shown to contract these fibers and generate their production [Zelickson BD, Kist D, Bernstein E, Brown DB, Ksenzenko S, Burns J, Kilmer S, Mehregan D, Pope K. Histological and ultrastructural evaluation of the effects of a radiofrequency-based nonablative dermal remodeling device: a pilot study. Arch Dermatol. 2004 Feb; 140(2):204-9], methods of differentially heating the dermis (deep tissue diathermy) have recently arisen.

A unique method of treating the dermis is called Pulsed Electromagnetic Fields (PEMF) therapy. This method usually employs electromagnetic radiation of different frequencies, - ranging from static magnetic fields, through extremely low frequencies to higher radiofrequencies (RF) - administered in pulses.

PEMF works in more than one way. The radiation absorbed by the tissue can heat the tissue to a desired temperature, depending on the power applied, the frequency transmitted, and, more importantly, the tissue characteristics. For example, the tissue can be heated to denaturation temperatures, which cause tissue damage and coagulation necrosis. Tissue can also be heated to lower temperatures, which can cause the aforementioned contraction of collagen fibers.

Another modus operandi involves non-thermal effects, which rely on the reaction of specific tissue components to characteristics of the applied radiation. These effects can be due to large charged molecules and their reaction to various frequencies and their harmonics, charged small ions in the cell membranes affecting cell function and reactions to hormones and chemical signals, charged small ions in the extracellular space and other poorly understood mechanisms.

Furthermore, applying the radiation in pulses was also found to have non-thermal effects. Yet more, specific combinations of frequency, duty cycle and transmitted power can cause specific tissue responses. Recent scientific research has determined PEMF characteristics which can cause desired biophysical responses.

It is now commonly accepted that weak electromagnetic fields (EMF) administered in pulses are capable of initiating various healing processes in fractures, multiple sclerosis and Parkinson's disease, and even delivering pain relief; however it seems that most of the conditions that seem most likely to respond to PEMF are musculoskeletal. Two decades ago, the FDA allowed the use of pulsed radiofrequency electromagnetic fields for treatment of pain and edema in superficial soft tissues. [Rosch, P.J., Markov, M.S., eds. Bioelectromagnetic Medicine, 2004; Marcel Dekker, NY, 251- 264].

PEMF can also be used for tissue rejuvenation or revitalization as described above. Several studies have addressed the effect of PEMF on dermal components. For example, *in vivo* trials showed that pulsed electromagnetic fields of certain field intensities and frequencies increased epidermal collagen synthesis [Ahmadian S, Zarchi SR, Bolouri B. Effects of extremely-low-frequency pulsed electromagnetic fields on collagen synthesis in rat skin. Biotechnol Appl Biochem. 2006 Feb; 43(Pt 2):71-75]. This newly-formed collagen increases skin elasticity and rejuvenates the appearance of the skin.

*In vitro* trials showed that PEMF increased the degree of endothelial cell tubulization and proliferation, and augmented angiogenesis primarily by stimulating endothelial release of FGF-2, inducing paracrine and autocrine changes in the surrounding tissue [Tepper OM et al. Electromagnetic fields increase in vitro and in vivo angiogenesis through endothelial release of FGF-2. FASEB J. 2004 Aug; 18(11):1231-3. Epub 2004 Jun 18]. Angiogenesis, the generation of new blood vessels, increases blood flow to the tissue, which in turn increases delivery of oxygen and nutritional substances to the tissue. This effect is most beneficial for injured tissue, promoting rapid and improved healing. The growth factor which is released further enhances the healing process, both in quality and speed of improvement.

The scientific evidence of the effect of PEMF on tissues was utilized in various applications. For example, US20050182462A1 discloses healthy deep tissue heating using PEMF for the purpose of causing contraction and tightening of the skin.

PEMF has also been used to improve skin wound healing. For example, WO08064272 discloses a method of treating a severe diabetic ulcer using PEMF. The patent also discloses the addition of intermittent compression therapy (ICT) and the use of low intensity ultrasound (up to 50 W/cm²), the latter aimed at inhibiting microbial growth.

Other methods of heating the dermis used non pulsating RF radiation, applied by antenna or electrodes. For example, WO98005380 discloses a method of tightening skin using an RF electromagnetic energy delivery device.

Improving the results of skin tightening based on dermis diathermy is still a long felt need, both for esthetic and therapeutic purposes.

WO2010/007614 A2 provides a system for increasing skin rejuvenation of a region of a patient's skin comprising a pulsed electromagnetic field (PEMF) frequency generator for constantly providing electromagnetic pulses to said region of a patient's skin and a deep tissue diathermy device for constantly applying heat to said region of a patient's skin up to temperature T. The system is adapted for simultaneously applying heat and PEMF to the region of a patient's skin, wherein application of the system increases skin rejuvenation such that the skin rejuvenation increase (SRI) is greater than the sum of the SRI provided by electromagnetic pulses increase and the SRI provided by the deep tissue diathermy device increase.

EP2564894 A1 describes a device for cosmetic improvement of the skin.

WO2011/034986 A2 describes a vaginal remodeling device and methods.

### SUMMARY OF THE INVENTION

According to the present invention, an integrated system is provided in accordance with claim 1. Preferred embodiments are defined in the dependent claims. The methods disclosed herein do not form part of the claimed invention as such, but can nevertheless be useful for understanding the operation and intended use of the system according to the invention.

### SUMMARY OF THE DISCLOSURE

It is an object of the present disclosure to provide a device for rejuvenating at least one region of mucosal tissue, comprising:
a pulsed electromagnetic frequency generator to apply pulsed electromagnetic field therapy to said region of mucosal tissue; and
a plurality of electrodes in communication with an RF tissue diathermy device wherein said said RF tissue diathermy device, via said electrodes, heats said at least one region of mucosal tissue.

It is another object of the present disclosure to provide the device as disclosed above, wherein at least one of the following is being held true:
a. at least a portion of said at least one region of mucosal tissue is at least temporarily maintainable at a predetermined temperature range T₁ while another at least a portion of said at least one region of said mucosal tissue is at least temporarily maintainable at predetermined temperature range T₂, where T₁ is equal to or different from T₂;
b. said device additionally comprises at least one temperature sensor adjacent to at least one of said plurality of electrodes;
c. said at least one region of mucosal tissue is within a vagina; and
d. said device additionally comprises a processor in communication with said at least one temperature sensor and with said database, said at least one temperature sensor is selected from a group consisting of: a thermistor, a thermocouple and any combination thereof.

It is another object of the present disclosure to provide the device as disclosed above, wherein at least one of the following is being held true:
a. said processor is configured to feedback control RF output to said pulsed electromagnetic frequency generator and said RF tissue diathermy device such that said temperature profile is maintainable in at least one predetermined region outside said device; and,
b. each said at least one temperature is measurable in said at least one portion of said region of mucosal tissue.

It is another object of the present disclosure to provide the device as disclosed above, wherein at least one of the following is being held true:
a. a duration of a pulse from said pulsed electromagnetic frequency generator is in a range between about 3 ms and about 1000 ms;
b. a frequency F of said pulses from said pulsed electromagnetic frequency generator is in a range between about 1 Hz and about 50 Hz; and
c. pulsed electromagnetic power from said pulsed electromagnetic frequency generator is configured to stimulate collagen production.

It is another object of the present disclosure to provide the device as disclosed above, wherein at least one of the following is being held true:
a. said pulsed electromagnetic power is configured to induce angiogenesis; and
b. said pulsed electromagnetic power comprises pulses at a frequency F of about 15 Hz, and a magnetic field intensity B of about 12 gauss.

It is another object of the present disclosure to provide the device as disclosed above, wherein a member of a group selected from: said at least one temperature in said temperature profile, said predetermined temperature range T₁, said predetermined temperature range T₁ and any combination thereof is in a range selected from a group consisting of: from about 30 degrees C and about 80 degrees C; and from about 40 degrees C to about 50 degrees C.

It is another object of the present disclosure to provide an integrated system for increasing rejuvenation of at least one region of a patient's mucosal tissue, said system comprising:
at least two electrodes configured to be placed on at least one said region of said patient's mucosal tissue;
an electromagnetic field generator configured to generate electromagnetic field pulses and to apply said electromagnetic field pulses to said at least one region of a patient's mucosal tissue; and,
a control system;
wherein:
   none of said electrodes is configured to penetrate said at least one region of a patient's mucosal tissue;
   each of said electrodes is configured to conduct RF pulses to at least one said region of said patient's mucosal tissue; said electrodes are configured to heat said mucosal tissue up to a temperature T in a range of 30 degrees Celsius to 80 degrees Celsius; and
   said control system is configured to control said electromagnetic field generator and application of said RF pulses by said electrodes.

It is another object of the present disclosure to provide the integrated system as disclosed above, wherein at least one of the following is held true:
a. at least one portion of said at least one region of said mucosal tissue is at least temporarily maintainable at a predetermined temperature range T₁ while at least one other portion of said at least one region of said mucosal tissue is at least temporarily maintainable at predetermined temperature range T₂, where T₁ is different from or the same as T₂;
b. said at least one mucosal tissue region is within a vagina; and
c. said system additionally comprises a database configured to store at least one temperature profile, said temperature profile comprising at least one temperature and at least one predetermined portion of said at least one region of mucosal tissue.

It is another object of the present disclosure to provide the integrated system as disclosed above, wherein at least one of the following is being held true:
a. said system additionally comprises a processor in communication with said database and with at least one temperature sensor;
b. said control system (6) monitors physical tissue parameters and changes a member of a group consisting of said applied heat, said electromagnetic pulses and any combination thereof accordingly;
c. a duration of each pulse applied by said system ranges between about 3 ms and about 1000 ms;
d. frequency F applied by the electromagnetic pulses of said system ranges between about 1 Hz and about 50 Hz;
e. frequency applied by the RF pulses of said system ranges between about 200 kHz and about 10 MHz; and
f. power P applied by said RF of said system ranges between about 1 W and about 100 W of RMS average power.

It is another object of the present disclosure to provide the integrated system as disclosed above, wherein at least one of the following is being held true:
a. each said at least one temperature sensor is configured to measure at least one temperature in said predetermined portion of said at least one mucosal region of said patient; and
b. said at least one temperature sensor is selected from a group consisting of: a thermistor, a thermocouple and any combination thereof.

It is another object of the present disclosure to provide a system (10) configured to increase rejuvenation of at least one region of a patient's mucosal tissue by synergistic application of heat and a pulsed electromagnetic field (PEMF) to at least one said region of a patient's mucosal tissue, said system comprising:
a pulsed electromagnetic field generator for generating a pulsed electromagnetic field (PEMF) frequency (2) for providing electromagnetic pulses to at least one said region of said patient's mucosal tissue according to a predetermined protocol; and
an RF generator configured to operate a plurality of electrodes, said operation of said electrodes configured to induce tissue diathermy;
wherein said system is configured to provide rejuvenation of at least one said region of mucosal tissue via a synergistic combination of said PEMF and said tissue diathermy,
further wherein said plurality of electrodes (41) are configured to be placeable on said at least one region of said patient's mucosal tissue.

It is another object of the present disclosure to provide the system as disclosed above, wherein at least one of the following is being held true:
a. at least one said region of mucosal tissue is within a vagina;
b. said system additionally comprises at least one temperature sensor, said at least one temperature sensor is selected from a group consisting of: a thermistor, a thermocouple and any combination thereof;
c. at least one portion of said at least one region of said mucosal tissue is at least temporarily maintainable at a predetermined temperature range T₁ while at least one other portion of said at least one region of said mucosal tissue is at least temporarily maintainable at predetermined temperature range T₂, where T₁ is different from or the same as T₂;
d. said system additionally comprises a database configured to store at least one temperature profile, said temperature profile comprising at least one temperature and at least one portion of said mucosal tissue region;
e. a control system (**6**) includes a cooling mechanism for cooling said region of mucosal tissue; and
f. said system (**10**) is encased in at least one platform.

It is another object of the present disclosure to provide the system as disclosed above, wherein at least one of the following is being held true:
a. said system additionally comprises a processor in communication with said at least one temperature sensor and with said database; and
b. a member of a group consisting of said temperature, said temperature range T₁, said temperature range T₂ and any combination thereof is higher than about 30 degrees Celsius and lower than about 80 degrees Celsius.

It is another object of the present disclosure to provide the system as disclosed above, wherein at least one of the following is being held true:
a. said processor is configured to feedback control said pulsed electromagnetic frequency generator and said RF generator such that said temperature profile is maintainable in at least one predetermined region outside said device; and,
b. each said at least one temperature is measurable in said at least one portion of said region of mucosal tissue.

It is another object of the present disclosure to provide the system as disclosed above, wherein all said electrodes are configured to simultaneously apply said RF power to said region of mucosal tissue.

It is another object of the present disclosure to provide a method (**400**) of increasing mucosal tissue rejuvenation of at least one region of a patient's mucosal tissue, comprising steps of:
a. obtaining (i) a pulsed electromagnetic frequency generator; and (ii) an RF tissue diathermy device; and,
b. applying, either simultaneously or in batch like manner, (a) heat to at least a portion of mucosal tissue within said at least one region of mucosal tissue up to temperature T; (b) pulses of electromagnetic field to at least a portion of said at least one region of mucosal tissue; and any combination thereof
wherein said increase in said rejuvenation of said at least one region of mucosal tissue is greater than the sum of increase in rejuvenation due to said applying heat to said mucosal tissue and increase due to said applying pulses electromagnetic therapy to said at least one region of mucosal tissue..

It is another object of the present disclosure to provide the method as disclosed above, additionally comprising at least one of the following steps:
a. at least temporarily maintaining at least a portion of said at least one region of mucosal tissue at a predetermined temperature range T₁ while at least temporarily maintaining at least one other portion of said region of mucosal tissue at predetermined temperature range T₂, where T₁ is the same as or different from T₂;
b. selecting said region of mucosal tissue to be within a vagina;
c. a step selected from a group consisting of: simultaneously applying said pulsed electromagnetic field and said heat, sequentially applying said pulsed electromagnetic field and said heat and any combination thereof;
d. selecting at least one parameter from a group consisting of (a) the duration of each pulse applied by said pulsed electromagnetic frequency generator (2) is greater than about 3 ms and lower than about 1000 ms; (b) the magnetic field intensity B of each pulse applied by said step of applying pulsed electromagnetic therapy to said region is in a range from 0 to 15 Gauss; (c) the frequency F applied by the pulses applied by said step of applying pulsed electromagnetic therapy to said region is higher than about 1 Hz and lower than about 50 Hz; (d) the power P applied by said step of applying pulsed electromagnetic therapy to said region is greater than about 1 W and lower than about 100 W of RMS average power and any combination thereof;
e. selecting a duration for application of said heat is in a range from about 0.01 minutes to about 60 minutes;
f. selecting a manner of applying said heat and said pulsed electromagnetic therapy from a group consisting of: simultaneously, sequentially, separately and any combination thereof; and
g. said step of applying heat to a tissue comprises steps of:
   i. electrically coupling at least two electrodes to said RF tissue diathermy device;
   ii. placing said at least two electrodes on said at least one region of mucosal tissue;
      and,
   iii. applying a member of a group consisting of: said electromagnetic pulses, said RF power via at least one of said electrodes, and any combination thereof to said at least one region of mucosal tissue.

It is another object of the present disclosure to provide the method as disclosed above, additionally comprising at least one of the following sets of steps:
a. a set of steps comprising: (a) providing a database comprising at least one temperature profile, said temperature profile containing at least one temperature and at least one predetermined region of mucosal tissue; (b) measuring said at least one temperature at said at least one predetermined region of mucosal tissue; and (c) feedback controlling RF output to said pulsed electromagnetic frequency generator and said RF tissue diathermy device, thereby maintaining said temperature profile in said at least one predetermined region.
b. a set of steps comprising: (a) providing a vaginal treatment device comprising a distal portion and a proximal portion, said distal portion and said proximal portion reversibly connectible; said proximal portion in communication with a member of a group consisting of said pulsed electromagnetic frequency generator, said RF tissue diathermy device and any combination thereof; said distal portion comprising at least two electrodes; (b) placing said distal portion at least partially within said vagina; (c) keeping at least a part of said distal portion substantially stationary within said vagina for a time period in a range between about 1 minute and about 20 minutes; (d) for at least a portion of said time period, activating a member of a group consisting of said pulsed electromagnetic frequency generator, said RF tissue diathermy device and any combination thereof, said activation applying a member of a group consisting of pulsed electromagnetic field, heat and any combination thereof to tissue in said vagina; and (e) for at least a portion of said time period, measuring temperature of said tissue in said vagina.

It is another object of the present disclosure to provide the method as disclosed above, additionally comprising steps of (a) providing said distal portion with at least two pairs of electrodes; and (b) controlling each pair separately, thereby maintaining said temperature profile within at least a portion of said vagina.

It is another object of the present disclosure to provide an integrated system for increasing rejuvenation of at least one region of a patient, said at least one region selected from a group consisting of said patient's skin, said patient's mucosal tissue and any combination thereof, said system comprising:
at least two electrodes configured to be placed on at least one said region of said patient;
an electromagnetic field generator configured to generate electromagnetic field pulses;
a generator configured to generate RF power; and
a control system;
wherein:
   none of said at least two electrodes is configured to penetrate said at least one region;
   each of said at least two electrodes is configured to provide RF pulses to at least one said region, said RF power configured to apply heat up to a temperature T in a range of about 30 degrees C to about 80 degrees C;
   said control system is configured to control a member of a group consisting of: said electromagnetic field generator, application of said heat by said electrodes and any combination thereof; and,
   said electromagnetic pulses are applyable to said at least one region and said heat is applyable to said at least one region in a manner selected from a group consisting of: simultaneously, sequentially, separately and any combination thereof.

It is another object of the present disclosure to provide the integrated system as disclosed above, wherein at least one of the following is held true:
a. at least one portion of said at least one region is at least temporarily maintainable at a predetermined temperature range T₁ while at least one other portion of said at least one region is at least temporarily maintainable at predetermined temperature range T₂, where T₁ is different from or the same as T₂;
b. said at least one region of said patient is within a vagina;
c. said system additionally comprises a database configured to store at least one temperature profile, said temperature profile comprising at least one temperature and at least one predetermined region of said patient;
d. said heat applied to said region of said patient is obtainable by emitting RF radiation or via producing electrical current absorbed by tissue;
e. said system is configured to provide a dynamic magnetic field such that a magnitude of said electromagnetic pulses are variable with time;
f. physical tissue parameters are monitorable by said control system (6) and a member of a group consisting of: said applied heat, said electromagnetic pulses and any combination thereof is changeable accordingly;
g. a duration of each pulse applied by said system ranges between about 3 ms and about 1000 ms;
h. frequency F applied by the pulses of said system ranges between about 200 kHz and about 1MHz; and
i. power P applied by the pulses of said system ranges between about 1 W and about 100 W of RMS average power.

It is another object of the present disclosure to provide the integrated system as disclosed above, additionally comprising a processor in communication with said database and with at least one temperature sensor, said at least one temperature sensor configured to measure at least one temperature in at least one portion of said at least one region of said patient; said at least one temperature sensor is selected from a group consisting of: a thermistor, a thermocouple and any combination thereof.

It is another object of the present invention to provide a device for rejuvenating at least one region of mucosal tissue, comprising:
a pulsed electromagnetic frequency generator;
an RF tissue diathermy device; and
a plurality of electrodes in communication with said pulsed electromagnetic frequency generator and said RF tissue diathermy device;
wherein said pulsed electromagnetic frequency generator and said RF tissue diathermy device, via said electrodes, heat said at least one region of mucosal tissue
further wherein at least a portion of said at least one region of mucosal tissue is at least temporarily maintainable at a predetermined temperature range T₁ while another at least a portion of said at least one region of said mucosal tissue is at least temporarily maintainable at predetermined temperature range T₂, where T₁ is equal to or different from T₂.

It is another object of the present disclosure to provide the device, additionally comprising at least one temperature sensor adjacent to at least one of said plurality of electrodes.

It is another object of the present invention to provide the device, wherein said at least one temperature sensor is selected from a group consisting of: a thermistor, a thermocouple and any combination thereof.

It is another object of the present disclosure to provide the device, wherein said at least one region of mucosal tissue is in a vagina.

It is another object of the present disclosure to provide the device, additionally comprising a database configured to store at least one temperature profile, said temperature profile comprising at least one temperature and at least one portion of said at least one region of mucosal tissue.

It is another object of the present disclosure to provide the device, additionally comprising a processor in communication with said at least one temperature sensor and with said database.

It is another object of the present disclosure to provide the device, wherein said processor is configured to feedback control output to said pulsed electromagnetic frequency generator and said RF tissue diathermy device such that said temperature profile is maintainable in at least one predetermined region outside said device.

It is another object of the present disclosure to provide the device, wherein each said at least one temperature is measurable in at least one portion of said at least one region of mucosal tissue.

It is another object of the present disclosure to provide the device, wherein a pulse shape of said pulsed electromagnetic power is selected from a group consisting of: square wave, triangular wave, sawtooth wave, ramp wave, spiked wave and any combination thereof.

It is another object of the present invention to provide the device, wherein a duration of a pulse of said pulsed electromagnetic power is in a range between about 3 ms and about 1000 ms.

It is another object of the present disclosure to provide the device, wherein a frequency F of said pulses of said pulsed electromagnetic power is in a range between about 1 Hz and about 1 MHz.

It is another object of the present disclosure to provide the device, wherein said pulsed electromagnetic power is configured to induce angiogenesis.

It is another object of the present disclosure to provide the device, wherein said pulsed electromagnetic power comprises pulses at a frequency F of about 15 Hz, a pulse duration of about 5 ms and a magnetic field intensity B of about 12 gauss

It is another object of the present disclosure to provide the device, wherein said pulsed electromagnetic power is configured to stimulate collagen production.

It is another object of the present disclosure to provide the device, wherein said pulsed electromagnetic power comprises pulses at a frequency F between about 10 Hz and about 25 Hz and intensity I of about 20 gauss.

It is another object of the present disclosure to provide the device, wherein said at least one temperature in said temperature profile is in a range from about 30 degrees C and about 80 degrees C.

It is another object of the present disclosure to provide the device, wherein said at least one temperature in said temperature profile is in a range from about 40 degrees C to about 50 degrees C.

It is another object of the present disclosure to provide the device, wherein pulsed electromagnetic power from said pulsed electromagnetic frequency generator and heat from said RF tissue diathermy device synergistically rejuvenate at least one region of mucosal tissue such that rejuvenation by means of said pulsed electromagnetic power and said heat is greater than the sum of rejuvenation by means of said pulsed electromagnetic power alone and said heat alone.

It is another object of the present disclosure to provide the device, additionally comprising at least one suction unit, said at least one suction unit in fluid communication with at least one port in said device, said port configured to allow passage of air therethrough.

It is another object of the present disclosure to provide the device, wherein said port is configured to draw said tissue into contact with said device.

It is another object of the present disclosure to provide the device, wherein said suction unit is configured to operate either continuously or in pulses.

It is another object of the present disclosure to provide an integrated system for increasing rejuvenation of at least one region of a patient's mucosal tissue, said system comprising:
at least two electrodes configured to be placed on said at least one region;
an electromagnetic field generator configured to generate electromagnetic field pulses; and,
a control system;
wherein:
   none of said at least two electrodes is configured to penetrate said at least one region;
   each of said at least two electrodes is configured to provide electromagnetic pulses to said at least one region;
   each of said at least two electrodes is configured to apply heat up to a temperature T in a range of about 30 degrees Celsius to about 80 degrees Celsius;
   said control system is configured to control said electromagnetic field generator and application of said heat by said at least two electrodes; and,
   each of said at least two electrodes is configured to provide said electromagnetic pulses to said at least one region and to apply said heat to said at least one region, said providing of said electromagnetic pulses and said application of heat occurring in a manner selected from a group consisting of simultaneously, sequentially, separately and any combination thereof.

It is another object of the present disclosure to provide the system, wherein at least one portion of said at least one region is at least temporarily maintainable at a predetermined temperature range T₁ while at least one other portion of said at least one region is at least temporarily maintainable at predetermined temperature range T₂, where T₁ is different from or the same as T₂.

It is another object of the present disclosure to provide the system, wherein said at least one region is in a vagina.

It is another object of the present disclosure to provide the system, additionally comprising a database configured to store at least one temperature profile, said temperature profile comprising at least one temperature and at least one predetermined portion of said at least one region.

It is another object of the present disclosure to provide the system, additionally comprising at least one temperature sensor adjacent to at least one of said plurality of electrodes.

It is another object of the present disclosure to provide the system, wherein said at least one temperature sensor is selected from a group consisting of: a thermistor, a thermocouple and any combination thereof.

It is another object of the present disclosure to provide the system, additionally comprising a processor in communication with said at least one temperature sensor and with said database.

It is another object of the present disclosure to provide the system, wherein said processor is configured to feedback control output to said pulsed electromagnetic frequency generator and said RF tissue diathermy device such that said temperature profile is maintainable in at least one predetermined region outside said device.

It is another object of the present disclosure to provide the system, wherein each said at least one temperature is measurable in saidat least one predetermined portion of said at least one region of mucosal tissue.

It is another object of the present disclosure to provide the system, wherein said at least one temperature sensor is selected from a group consisting of: a thermistor, a thermocouple and any combination thereof.

It is another object of the present disclosure to provide the system, wherein said heat applied to said at least one region of a patient's mucosal tissue is obtained by emitting RF radiation or via producing electrical current absorbed by subcutaneous tissue.

It is another object of the present disclosure to provide the system, wherein said electromagnetic pulses are selected from a waveform with a frequency between about 10 Hz and about 25 Hz and intensity of about 20 gauss or a waveform with a frequency of about 15 Hz, duration of about 5 milliseconds and intensity of about 12 gauss.

It is another object of the present disclosure to provide the system, wherein said system is configured to provide a dynamic magnetic field such that said electromagnetic pulses vary with time.

It is another object of the present disclosure to provide the system, wherein each of said electromagnetic pulses has a shape selected from the group consisting of a square wave, a sine wave, a triangular wave, a sawtooth wave, a ramp wave, a spiked wave and any combination thereof.

It is another object of the present disclosure to provide the system, wherein said control system (6) monitors physical tissue parameters and changes said applied heat and said electromagnetic pulses accordingly.

It is another object of the present disclosure to provide the system, wherein a duration of each pulse applied by said system ranges between about 3 and about 1000 milliseconds.

It is another object of the present disclosure to provide the system, wherein a frequency F applied by the pulses of said system ranges between about 1 Hz and about 1 MHz.

It is another object of the present disclosure to provide the system, wherein power P applied by the pulses of said system ranges between about 1 W and about 100 W power per pulse.

It is another object of the present disclosure to provide the system, additionally comprising at least one suction unit, said at least one suction unit in fluid communication with at least one port in said system, said port configured to allow passage of air therethrough.

It is another object of the present disclosure to provide the system, wherein said port is configured to draw said tissue into contact with said system.

It is another object of the present disclosure to provide the system, wherein said suction unit is configured to operate either continuously or in pulses.

It is another object of the present disclosure to provide a system (10) configured to increase rejuvenation of at least one region of a patient's mucosal tissue by synergistic application of heat and a pulsed electromagnetic field (PEMF) to said region, said system comprising:
a. a pulsed electromagnetic field generator for generating a pulsed electromagnetic field (PEMF) frequency (2) for providing electromagnetic pulses to said at least one region according to a predetermined protocol; and
b. an RF generator configured to operate a plurality of electrodes;

wherein said system is configured to provide rejuvenation of said region via a synergistic combination of PEMF and RF tissue diathermy,
wherein said plurality of electrodes (41) are configured to be placeable on said region.

It is another object of the present disclosure to provide the system, additionally comprising at least one temperature sensor.

It is another object of the present disclosure to provide the system, wherein said at least one temperature sensor is selected from a group consisting of: a thermistor, a thermocouple and any combination thereof.

It is another object of the present disclosure to provide the system, wherein at least one least one portion of said at least one region is at least temporarily maintainable at a predetermined temperature range T₁ while least one other portion of said at least one region is at least temporarily maintainable at predetermined temperature range T₂, where T₁ is different from or the same as T₂.

It is another object of the present disclosure to provide the system, wherein each said at least one temperature is measurable in said at least one portion of said at least one region.

It is another object of the present disclosure to provide the system, wherein said at least one region is in a vagina.

It is another object of the present disclosure to provide the system, wherein said predetermined protocol is selected from a group consisting of: a series of pulses with a frequency between about 10 Hz and about 25 Hz and an intensity of about 20 gauss; a series of pulses of duration of about 5 ms and intensity of about 12 gauss, repeated at a frequency of about 15 Hz; and, any combination thereof.

It is another object of the present disclosure to provide the system, wherein each of said plurality of electrodes is configured to both (i) provide electromagnetic pulses to said at least one region; and, *(ii)* apply heat up to a temperature of about 80 degrees Celsius to said at least one region.

It is another object of the present disclosure to provide the system, wherein each of said plurality of electrodes is configured to provide said electromagnetic pulses to said at least one region and apply heat to said at least one region simultaneously, sequentially, separately and any combination thereof.

It is another object of the present disclosure to provide the system, additionally comprising a database configured to store at least one temperature profile, said temperature profile comprising at least one temperature and at least one predetermined portion of said at least one region.

It is another object of the present disclosure to provide the system, additionally comprising a processor in communication with said at least one temperature sensor and with said database.

It is another object of the present disclosure to provide the system, wherein said processor is configured to feedback control output to said pulsed electromagnetic frequency generator and said RF generator such that said temperature profile is maintainable in at least one predetermined region outside said device.

It is another object of the present disclosure to provide the system, wherein said RF generator is an RF tissue diathermy device.

It is another object of the present disclosure to provide the system, wherein said RF tissue diathermy device (4) is selected from a group consisting of: an electrical current producing means configured for producing electrical current absorbed by subcutaneous tissue, electromagnetic induction, and direct heat applying instrument.

It is another object of the present disclosure to provide the system, wherein said RF generator **(4)** additionally comprises:
a. at least one electrical output device configured to generate either RF electromagnetic power or electrical current; and
b. an electrical coupling between said at least one electrical output device and said plurality of electrodes such that said plurality of electrodes are configured to simultaneously apply a member of a group consisting of: said RF power, said electrical current and any combination thereof to said region.

It is another object of the present disclosure to provide the system, wherein said pulsed electromagnetic frequency generator is configured to provide a dynamic magnetic field such that said electromagnetic pulses vary with time.

It is another object of the present disclosure to provide the system, wherein at least one of the following is being held true (a) the shape of said electromagnetic pulse is selected from the group consisting of a square wave, a sine wave, a triangular wave, a sawtooth wave, a ramp wave, a spiked wave and any combination thereof; (b) the duration of each pulse applied by said pulsed electromagnetic frequency generator (2) ranges between about 3 ms and about 1000 ms; (c) the frequency applied by the pulses of said pulsed electromagnetic frequency generator (2) ranges between about 1 Hz and about 1 MHz; (d) the power applied by the pulses of said pulsed electromagnetic frequency generator (2) ranges between about 1 W and about 100 W per pulse and any combination thereof.

It is another object of the present disclosure to provide the system, wherein said temperature is higher than about 30 degrees Celsius and lower than about 80 degrees Celsius.

It is another object of the present disclosure to provide the system, wherein a power supply and control system (6) includes a cooling means for cooling said at least one region.

It is another object of the present disclosure to provide the system, wherein said system (10) is encased in at least one platform.

It is another object of the present disclosure to provide the system, wherein said pulsed electromagnetic frequency generator (2) and said RF tissue diathermy device (4) have more than one applicator to treat more than one region simultaneously; further wherein said pulsed electromagnetic frequency generator (2) has electrostatic shielding.

It is another object of the present disclosure to provide the system, wherein said system is configured to operate according to an IEC protocol selected from the group consisting of IEC 60601-2-33, IEC 60601-2-3, IEC 60601-1-8, IEC 60601-1-6, IEC 60601-1-4, IEC 60601-1-2, IEC 60601-1-1, and any combination thereof.

It is another object of the present disclosure to provide the system, additionally comprising at least one suction unit, said at least one suction unit in fluid communication with at least one port in said system, said port configured to allow passage of air therethrough.

It is another object of the present disclosure to provide the system, wherein said port is configured to draw said tissue into contact with said system.

It is another object of the present disclosure to provide the system, wherein said suction unit is configured to operate either continuously or in pulses.

It is another object of the present disclosure to provide a method (400) of increasing rejuvenation of at least one region of a patient's mucosal tissue, comprising steps of:
a. obtaining (i) a pulsed electromagnetic frequency generator; and *(ii)* an RF tissue diathermy device; and,
b. applying either simultaneously or in a batch like manner (a) heat to a subcutaneous tissue within at least a portion of said at least one region up to temperature T; or (b) pulses of electromagnetic field to at least a portion of said at least one region; and any combination thereof
wherein said increase in said rejuvenation of said at least one region is greater than the sum of increase in rejuvenation due to said applying heat to said subcutaneous tissue and increase in rejuvenation due to said applying said pulses electromagnetic therapy to said at least one region.

It is another object of the present disclosure to provide the method, additionally comprising step of at least temporarily maintaining at least one portion of said at least one region at a predetermined temperature range T₁ while is at least temporarily maintaining at least one other portion of said at least one region at predetermined temperature range T₂, where T₁ is different from or the same as T₂

It is another object of the present disclosure to provide the method, additionally comprising step of selecting said mucosal tissue to be in a vagina.

It is another object of the present disclosure to provide the method, additionally comprising step of providing at least one temperature sensor.

It is another object of the present disclosure to provide the method, additionally comprising step of selecting said at least one temperature sensor from a group consisting of: a thermistor, a thermocouple and any combination thereof.

It is another object of the present disclosure to provide the method, additionally comprising steps of: (a) providing a database comprising at least one temperature profile, said at least one temperature profile containing at least one temperature and at least one predetermined region; (b) measuring said at least one temperature at said at least one predetermined region; and (c) feedback controlling output to said pulsed electromagnetic frequency generator and said RF tissue diathermy device, thereby maintaining said temperature profile in said at least one predetermined region.

It is another object of the present disclosure to provide the method, additionally comprising at least one of the following steps: (i) reducing, by means of said system, ***side effects*** such that said reduction in said ***side effects*** is greater than a sum of reduction due to ***side effects*** of said electromagnetic pulses alone and reduction due to ***side effects*** of said RF tissue diathermy alone, and *(ii)* reducing, by means of said system, ***harmful effects*** such that said reduction in ***harmful effects*** is greater than a sum of reduction due to ***harmful effects*** of said electromagnetic pulses alone and ***harmful effects*** of said RF tissue diathermy alone.

It is another object of the present disclosure to provide the method, additionally comprising a step selected from a group consisting of: monitoring said steps of applying heat to a subcutaneous tissue within said at least one region, controlling said steps of applying heat to a subcutaneous tissue within said at least one region, monitoring said step of applying pulsed electromagnetic therapy to said at least one region, controlling said step of applying pulsed electromagnetic therapy to said at least one region, and any combination thereof.

It is another object of the present disclosure to provide the method, additionally comprising a step of applying a dynamic magnetic field to said at least one region.

It is another object of the present disclosure to provide the method, additionally comprising steps of:
a. storing in a communicable database predetermined parameters defining (i) safe treatment parameters and *(ii)* unsafe treatment parameters; said parameters are selected from a group consisting of: total duration time tₜ of said treatment, time during which pulses of electromagnetic field are applied tₚ, temperature T of said tissue, duty cycle tₚ/tₜ, frequency F, power P applied by the pulses of said pulsed electromagnetic frequency generator, depth D of said treated tissue, magnetic field intensity B, tissue impedance, specific absorption rate (SAR), treatment depth, superficial muscle contractions and any combination thereof;
b. sensing electromagnetic radiation and heat radiation parameters selected from a group consisting of total duration time tₜ of said treatment, time during which pulses of electromagnetic field are applied tₚ, temperature T of said tissue, duty cycle tₚ/tₜ, frequency F, power P applied by the pulses of said pulsed electromagnetic frequency generator, depth D of said treated tissue, magnetic field intensity B, tissue impedance, specific absorption rate (SAR), treatment depth, superficial muscle contractions and any combination thereof;
c. allowing said electromagnetic radiation and said heat radiation if said parameters within said safe treatment parameters and stopping said electromagnetic radiation and said heat radiation if said radiation parameters are in said unsafe treatment parameters.

It is another object of the present disclosure to provide the method, additionally comprising a step of selecting a shape of said electromagnetic pulse from a group consisting of: a square wave, a sine wave, a triangular wave, a sawtooth wave, a ramp wave, a spiked wave and any combination thereof.

It is another object of the present disclosure to provide the method, additionally comprising a step of applying at least one selected from a group consisting of (a) electromagnetic pulses at frequency between about 10 Hz and about 25 Hz and intensity of about 20 gauss; (b) a electromagnetic pulse at a frequency of about 15 Hz, duration of about 5 ms and intensity of about 12 gauss.

It is another object of the present disclosure to provide the method, additionally comprising a step of selecting said RF tissue diathermy device from a group consisting of: a device emitting RF radiation, a device inducing electromagnetic field within tissue and any combination thereof.

It is another object of the present disclosure to provide the method, wherein said step of applying heat to said subcutaneous tissue additionally comprises steps of:
a. obtaining at least one electrical output device configured to generate either RF electromagnetic power or electrical current;
b. electrically coupling at least two electrodes to said electrical output device;
c. placing said at least two electrodes on said region of mucosal tissue; and,
d. simultaneously applying via all said electrodes said RF power or said electrical current to said region of mucosal tissue.

It is another object of the present disclosure to provide the method, additionally comprising a step of selecting at least one parameter from a group consisting of (a) duration of each pulse applied by said pulsed electromagnetic frequency generator (2) is greater than about 3 ms and less than about 1000 ms; (b) frequency F applied by saidpulses applied by said step of applying pulsed electromagnetic therapy to said region is greater than about 1 Hz and less than about 1 MHz; (c) power P applied by said step of applying pulsed electromagnetic therapy to said at least one region is greater than about 1 W and less than about 100 W per pulse.

It is another object of the present disclosure to provide the method, wherein, during said step of applying heat, said heat is applied for a time greater than about 0.01 minutes and less than about 60 minutes.

It is another object of the present disclosure to provide the method, wherein said heat and said pulsed electromagnetic field are applied simultaneously, sequentially, separately and any combination thereof.

It is another object of the present disclosure to provide the method, wherein said step of applying heat is performed by a device selected from a group consisting of: RF tissue diathermy, electromagnetic induction, and any combination thereof.

It is another object of the present disclosure to provide the method, additionally comprising at least one suction unit, said at least one suction unit in fluid communication with at least one port in said system, said port configured to allow passage of air therethrough.

It is another object of the present disclosure to provide the method, additionally comprising step of, by means of said port and said suction unit, drawing said tissue into contact with said system.

It is another object of the present disclosure to provide the method, additionally comprising step of operating said suction unit either continuously or in pulses.

It is another object of the present disclosure to provide an integrated system for increasing rejuvenation of at least one region of a patient, said at least one region selected from a group consisting of said patient's skin, said patient's mucosal tissue and any combination thereof, said system comprising:
at least two electrodes configured to be placed on said at least one region;
an electromagnetic field generator configured to generate electromagnetic field pulses; and
a control system; wherein:
   none of said at least two electrodes is configured to penetrate said at least one region;
   each of said at least two electrodes is configured to provide electromagnetic pulses to said at least one region;
   each of said at least two electrodes is configured to apply heat up to a temperature T in a range of about 30 degrees C to about 80 degrees C;
   said control system is configured to control said electromagnetic field generator and application of said heat by said at least two electrodes; and,
   said electrodes are configured to provide said electromagnetic pulses to said at least one region and to apply said heat to said at least one region in a manner selected from a group consisting of: simultaneously, sequentially, separately and any combination thereof.

It is another object of the present disclosure to provide the system, wherein at least one one portion of said at least one region is at least temporarily maintainable at a predetermined temperature range T₁ while at least one other portion of said at least one region is at least temporarily maintainable at a predetermined temperature range T₂, where T₁ is different from or the same as T₂.

It is another object of the present disclosure to provide the system, wherein said at least one region is in a vagina.

It is another object of the present disclosure to provide the system, wherein at least one least one portion of said at least one region is at least temporarily maintainable at a predetermined temperature range T₁ while at least one other portion of said at least one region is at least temporarily maintainable at predetermined temperature range T₂, where T₁ is different from or the same as T₂.

It is another object of the present disclosure to provide the system, additionally comprising at least one temperature sensor.

It is another object of the present disclosure to provide the system, wherein said at least one temperature sensor is selected from a group consisting of: a thermistor, a thermocouple and any combination thereof.

It is another object of the present disclosure to provide the system, additionally comprising a processor in communication with said at least one temperature sensor and with said database.

It is another object of the present disclosure to provide the system, wherein said processor is configured to feedback control output to said pulsed electromagnetic frequency generator and said RF tissue diathermy device such that said temperature profile is maintainable in at least one predetermined region outside said device.

It is another object of the present disclosure to provide the system, additionally comprising a database configured to store at least one temperature profile, said temperature profile comprising at least one temperature and at least one predetermined portion of said region.

It is another object of the present disclosure to provide the system, wherein each said at least one temperature is measurable in said at least one portion of said region.

It is another object of the present disclosure to provide the system, wherein said heat applied to said at least one region is obtained by emitting RF radiation or via producing electrical current absorbed by subcutaneous tissue.

It is another object of the present disclosure to provide the system, wherein said electromagnetic pulses are selected from a group consisting of: a waveform with a frequency greater than about 10 Hz and less than about 25 Hz and an intensity of about 20 gauss; and a waveform with a frequency of about 15 Hz, a duration of about 5 ms and intensity of about 12 gauss.

It is another object of the present disclosure to provide the system, wherein said system is configured to provide a dynamic magnetic field such that average intensity of said electromagnetic pulses is variable with time.

It is another object of the present disclosure to provide the system, wherein each of said electromagnetic pulses has a shape selected from a group consisting of: a square wave, a sine wave, a triangular wave, a sawtooth wave, a ramp wave, a spiked wave and any combination thereof.

It is another object of the present disclosure to provide the system, wherein said control system (6) is configured to monitor physical tissue parameters and to change said applied heat and said electromagnetic pulses accordingly.

It is another object of the present disclosure to provide the system, wherein a duration of each pulse applied by said system is in a range between about 3 ms and about 1000 ms.

It is another object of the present disclosure to provide the system, wherein a frequency F of pulses applied by said system is in a range between about 1 Hz and about 1 MHz.

It is another object of the present disclosure to provide the system, wherein an RMS average power P applied by pulses of said system is in a range between about 1 W and about 100 W per pulse.

It is another object of the present disclosure to provide the system, additionally comprising at least one suction unit, said at least one suction unit in fluid communication with at least one port in said system, said port configured to allow passage of air therethrough.

It is another object of the present disclosure to provide the system, wherein said port is configured to draw said tissue into contact with said system.

It is another object of the present disclosure to provide the system, wherein said suction unit is configured to operate either continuously or in pulses.

### BRIEF DESCRIPTION OF THE DRAWINGS

In order to understand the invention and to see how it may be implemented in practice, a few preferred embodiments will now be described, by way of non-limiting example only, with reference to the accompanying drawings, in which:
Figs. 1A - 1D schematically present skin or mucosal tissue viability improving systems, comprising a pulsed electromagnetic frequency generator and a tissue diathermy device;
Fig. 2 schematically presents a skin viability improving system, comprising a pulsed electromagnetic frequency generator, a tissue diathermy device and a power supply and control system;
Figs. 3 - 8 schematically present methods of improving viability of skin or mucosal tissue;
Fig. 9 schematically illustrates a cross-section of an embodiment of a device for treating mucosal tissue in the vagina;
Fig. 10 schematically illustrates a temperature profile for an embodiment of a device for treating mucosal tissue in the vagina; and
Fig. 11A - 11F schematically illustrate embodiments of ports configured to allow suction to draw tissue into contact with the device.

### DETAILED DESCRIPTION

The following description is provided, alongside all chapters of the present invention, so as to enable any person skilled in the art to make use of said invention and sets forth the best modes contemplated by the inventor of carrying out this invention. Various modifications, however, will remain apparent to those skilled in the art, since the generic principles of the present invention have been defined specifically to provide means for at least temporarily rejuvenating mucosal tissue and for treating tissue lesions. Yet more the present invention provides means and system for tightening and rejuvenation of the vagina.

It is one object of the present invention to disclose a device used to at least temporarily rejuvenate mucosal tissues, such as vaginal tissues, by a synergistic approach of tissue diathermy, especially RF tissue diathermy, combined with application of PEMF, wherein at least two mechanisms of tissue rejuvenation are incorporated, one of them based on PEMF therapy. The latter improves the healing process initiated by the at least one tissue diathermy device.

The term **"Pulsed Electromagnetic Fields (PEMF)"** refers hereinafter in a non-limiting manner to electromagnetic radiation of different frequencies - ranging from static magnetic fields, through extremely low frequencies to radiofrequencies - administered in pulses.

The term **"Radio Frequency (RF)"** refers hereinafter in a non-limiting manner to part of the electromagnetic spectrum with frequency range of about 3 Hz to 300 GHz.

The term **"collagen"** refers hereinafter in a non-limiting manner to a long, fibrous structural protein which is a major component of the extracellular matrix that supports most tissues and gives cells structure. It is responsible for strength and elasticity of skin and mucosal tissue, and its degradation leads to the wrinkles and looseness that accompany aging.

The term **"epidermis"** refers hereinafter in a non-limiting manner to the outermost layer of the skin.

The term **"dermis"** refers hereinafter in a non-limiting manner to a layer of tissue beneath the epidermis or the mucosal surface that consists of connective tissue, and cushions the body from stress and strain.

The term **"deep tissue diathermy"** or **"tissue diathermy"** refers hereinafter in a non-limiting manner to a device which heats tissues beneath the epidermis or surface of the mucosal tissue.

The term **"electric diathermy"** refers hereinafter in a non-limiting manner to a device which uses high frequency alternating electric or magnetic fields, sometimes with no electrode or device contact to the skin or mucosal tissue, to induce gentle tissue heating by induction. For collagen fiber stimulation, typical electrical parameters may include, in a non-limiting manner, frequency of about 1 MHz, applied for about 6 seconds.

The term **"about"** refers hereinafter to a range of 25% below or above the referred value.

The term **"physical tissue parameters"** refers hereinafter to parameters such as tissue temperature, electric current, tissue impedance, specific absorption rate (SAR), treatment depth and superficial muscle contractions.

The term **"angiogenesis"** refers hereinafter to the generation of new blood vessels.

The term **"square wave"** refers hereinafter to a non-sinusoidal waveform named for its square shape.

The term **"triangular wave"** refers hereinafter to a non-sinusoidal waveform named for its triangular shape.

The term **"International Electrotechnical Commission Standards (IEC) 60601-1"** refers hereinafter to a medical electrical equipment standard. More specifically it refers to general requirements for basic safety and essential performance.

The term **"IEC 60601-1-1"** refers hereinafter to medical electrical equipment standard. More specifically it refers to general requirements for safety - Collateral standard: Safety requirements for medical electrical systems. The IEC 60601-1 set of standards are divided into three distinct areas. The first area is the basic standard IEC 60601-1. This is the general requirement for all electrical medical based products. The second area is the collateral standards, which cover across the board issues such as combining into a system with other devices, EMC, radiation protection, and programmable electronic medical systems (software, firmware, etc.). The standard numbers are IEC 60601-1-1, -1-2, -1-3, and -1-4 respectively.

The third area is the particular standards that deal with a specific type of medical device. The particular standards are identified as IEC 60601-2-XX where XX identifies the particular standard number for the particular type of medical equipment. An example would be IEC 60601-2-3 which is the particular standard for short-wave therapy equipment.

The term **"IEC 60601-1-2"** refers hereinafter to medical electrical equipment standard. More specifically it refers to general requirements for basic safety and essential performance - Collateral standard: Electromagnetic compatibility - Requirements and tests.

The term **"IEC 60601-1-3"** refers hereinafter to medical electrical equipment standard. More specifically it refers to general requirements for basic safety and essential performance - Collateral Standard: Radiation protection in diagnostic X-ray equipment.

The term **"IEC 60601-1-4"** refers hereinafter to medical electrical equipment standard. More specifically it refers to general requirements for safety - Collateral Standard: Programmable electrical medical systems.

The term **"IEC 60601-1-6"** refers hereinafter to medical electrical equipment standard. More specifically it refers to general requirements for basic safety and essential performance - Collateral standard: Usability.

The term **"IEC 60601-1-8"** refers hereinafter to medical electrical equipment standard. More specifically it refers to general requirements for basic safety and essential performance - Collateral Standard: General requirements, tests and guidance for alarm systems in medical electrical equipment and medical electrical systems.

The term **"IEC 60601-2-3"** refers hereinafter to medical electrical equipment standard. More specifically it refers to particular requirements for the safety of short-wave therapy equipment.

The term **"IEC 60601-2-9"** refers hereinafter to medical electrical equipment. More specifically it refers to particular requirements for the safety of patient contact dosemeters used in radiotherapy with electrically connected radiation detectors.

The term **"IEC 60601-2-29"** refers hereinafter to medical electrical equipment standard. More specifically it refers to particular requirements for the basic safety and essential performance of radiotherapy simulators.

The term "IEC 60601-2-33" refers hereinafter to medical electrical equipment standard.

More specifically it refers to particular requirements for the safety of magnetic resonance equipment for medical diagnosis.

The term "IEC 60601-2-35" refers hereinafter to medical electrical equipment standard. More specifically it refers to particular requirements for the safety of blankets, pads and mattresses intended for heating in medical use.

The present disclosure relates to physical therapeutic methods and systems. In such systems, a dynamic electromagnetic pulse and electromagnetic heating systems are incorporated together to accomplish physical therapy, especially tightening and rejuvenation of vaginal tissues.

The present disclosure provides a system configured to increase rejuvenation of a region of a patient's vaginal tissue. The system comprises in a non-limiting manner the following:
a. a pulsed electromagnetic field (PEMF) frequency generator (2) for constantly providing electromagnetic pulses to the region of the patient's vaginal tissues; and,
b. a tissue diathermy device **(4),** preferably an RF tissue diathermy device, applying heat to the region of the patient's vaginal tissues, thereby heating the patient's vaginal tissues to a temperature T.

The system **(10)** is configured for simultaneously apply heat and PEMF to said region of a patient's vaginal tissues. Furthermore, the system increases the tissue rejuvenation such that the increase is greater than the sum of the electromagnetic pulses increase and the tissue diathermy increase.

Furthermore the system reduces ***side effects*** and/or ***harmful effects*** of the electromagnetic pulses and/or said tissue diathermy such that the reduction of side effects and/or harmful effects is greater than the sum of the reduction of the effects of electromagnetic pulses and/or reduction of the effects of tissue diathermy.

It is another object of the present disclosure to provide an integrated system **(20)** configured to increase rejuvenation of a region of a patient's vaginal tissues. The system comprises at least two electrodes configured to be placed within a vagina, adjacent to a region of a patient's vaginal tissues. It is emphasized that each of the electrodes is configured for both (i) providing electromagnetic pulses to the region of a patient's vaginal tissues; and, *(ii)* applying heat up to temperature T to the region of a patient's vaginal tissues. Embodiments include: at least one of the electrodes is configured to simultaneously provide the electromagnetic pulses to the region of a patient's vaginal tissues; and, apply heat up to temperature T to the region of the patient's vaginal tissues; at least one of the electrodes is configured to sequentially provide the electromagnetic pulses to the region of a patient's vaginal tissues and, apply heat up to temperature T to the region of the patient's vaginal tissues, such that a pattern of application of electromagnetic pulses and application of heat is repeated at least once; and at least one of the electrodes is configured to separately provide the electromagnetic pulses to the region of a patient's vaginal tissues and, apply heat up to temperature T to the region of the patient's vaginal tissues, such that all of the electromagnetic pulses are provided in one time period and all of the application of heat is provided in another time period; with, in some variants, providing electromagnetic pulses coming first and, in other variants, application of heat coming first.

In some embodiments, during some time periods, none of the electrodes are active.

During a time period in which a plurality of electrodes is active, the following patterns of application can be used: all active electrodes provide the same treatment, whether it is simultaneously providing electromagnetic pulses and applying heat, providing electromagnetic pulses only or applying heat only; or at least one electrode is providing a treatment different from that provided by at least one other electrode.

The decrease in the side effects of the tissue diathermy results from the healing effects of the pulsed electromagnetic frequency therapy.

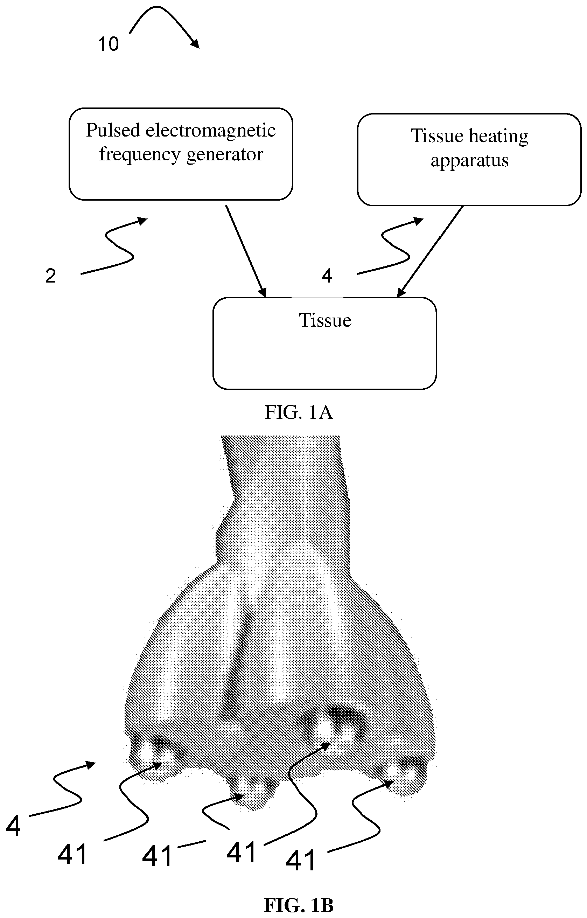

Reference is now made to **Figs. 1A** - **1D****,** illustrating a system **(10)** for increasing dermal tissue rejuvenation. As described above, and as shown in **Fig. 1A****,** the system comprises a pulsed electromagnetic frequency generator **(2)** for providing electromagnetic pulses to the region of a patient's dermal tissues; and, a tissue diathermy device **(4),** preferably an RF tissue diathermy device, configured to apply heat to the region of the patient's dermal tissues so as to heat the temperature of the tissues to a temperature T. It is emphasized that the system increases rejuvenation of the vaginal tissues such that the increase is greater than the sum of the rejuvenation increase due to the electromagnetic pulses increase and the rejuvenation increase due to the tissue diathermy.

By exposing the vaginal tissue to the combination of regulated heat and pulsed electromagnetic field, a *synergistic* effect of improving rejuvenation of the vaginal tissue is obtained.

The present invention relies on 2 effects, the thermal effect and the electromagnetic pulse effect:
**The thermal effect** includes heating the tissue to a temperature sufficiently high to produce tissue injury. Furthermore, when heat is generated within the dermis, it typically causes contraction and thickening of collagen fibers. Each of these will result in an overall tightened and rejuvenated appearance of the vaginal tissues.

Heat within the dermis creates a limited thermal injury. The body's natural response to this injury is to produce collagen at the site of the wound. This results in firmer, thicker, more elastic vaginal tissue. Usually the vaginal tissue is heated to temperatures below about 60 degrees C for short periods of time. The thermal effects can be produced by:
1. RF tissue diathermy;
2. Electrical means - by passing electrical current through the tissue;
3. Electromagnetic means - by transmitting electromagnetic fields to the tissue, by inducing (by means of electromagnetic induction) electromagnetic fields on the surface of the tissue, or a combination of these mechanisms;
and any combination thereof.

The **electromagnetic pulses** (either dynamic, with varying RMS average pulse intensity, or static, with constant RMS average pulse intensity) can start the natural healing processes which occur in response to an injury (especially angiogenesis and generation of new collagen fibers via the release of tissue growth factors).

The electromagnetic field generates movements of charged molecules (ions) within the intercellular fluids. This movement generates heat which can enhance the thermal effects discussed earlier.

It is acknowledged that healing is the process by which cells in the body regenerate and repair to reduce the size of a damaged area. Healing incorporates both the removal of necrotic tissue (demolition), and the replacement of this tissue.

The replacement can happen in two ways:
1. by regeneration: the necrotic cells are replaced by the same tissue as was originally there.
2. by repair: injured tissue is replaced with scar tissue.

The Pulsed Electromagnetic Fields (PEMF) applied by the system **(10),** as described above, have no direct thermal effects and rely on tissue components and their reactions to the applied radiation. These reactions to the applied radiation can be due to a response of large charged molecules to specific frequencies and harmonics of those frequencies, charged small ions in the cell membranes affecting cell function and affecting cell reactions to hormones and chemical signals, charged small ions in the extracellular space and other, poorly understood, mechanisms.

Furthermore, applying the radiation in pulses was also found to have non-thermal effects. Yet more, only specific combinations of frequency, duty cycle and transmitted power achieve specific tissue responses.

It is now commonly accepted that electromagnetic fields (EMF) or PEMF are capable of initiating various healing processes and for treatment of pain and edema in superficial soft tissues; two decades ago, the FDA allowed the use of pulsed radiofrequency electromagnetic fields for treatment of pain and edema in [Rosch, P.J., Markov, M.S., eds. Bioelectromagnetic Medicine, Marcel Dekker, 2004; NY, 251- 264].

The present invention utilizes PEMF combined with heating from a heat source which can be separate or can be integral to a device for rejuvenation and healing purposes as described above. The PEMF, as described above, when used at specific field intensities, duty cycles and frequencies, increases dermal collagen synthesis. This newly formed collagen increases tissue elasticity and, for skin treatment, rejuvenates the skin's appearance. Furthermore, PEMF increases the degree of endothelial cell tubulization and proliferation, and augments angiogenesis primarily by stimulating endothelial release of FGF-2, inducing paracrine and autocrine changes in the surrounding tissue. Angiogenesis, the generation of new blood vessels, increases blood flow to the tissue, which in turn increases oxygen and nutritional substances delivery to the tissue. This effect is most beneficial for injured tissue, promoting rapid and improved healing. The growth factor released further enhances the healing process, both in quality and in speed of improvement.

The following provides a more detailed description of the two combined effects.

As disclosed earlier, the present invention discloses a system (10) which incorporates both regulated heating means and electromagnetic pulses.

Typically, the heat can be produced by:
1. RF power
2. Electrical means - by passing electrical current.
3. Electromagnetic means - by transmitting or inducting (electromagnetic induction) electromagnetic field on the treated tissue. Reference is now made to Figs. 1B-1D, illustrating the system (10) according to some embodiments of the present invention.

According to these embodiments, the tissue diathermy device (4) comprises:
a. at least one electrical output device configured to generate electrical current; and,
b. at least two electrodes (41) electrically coupled to the electrical output device and placed on a region of the patient's skin or mucosal tissue.

According to some embodiments *all* of the electrodes are configured to *simultaneously* apply electrical current to the skin region. According to other embodiments, only a fraction of the electrodes are active at any given time.

**Fig. 1B** illustrates a system **(10)** for treating external skin tissue in which the tissue diathermy device **(4)** comprises 4 electrodes **(41).**

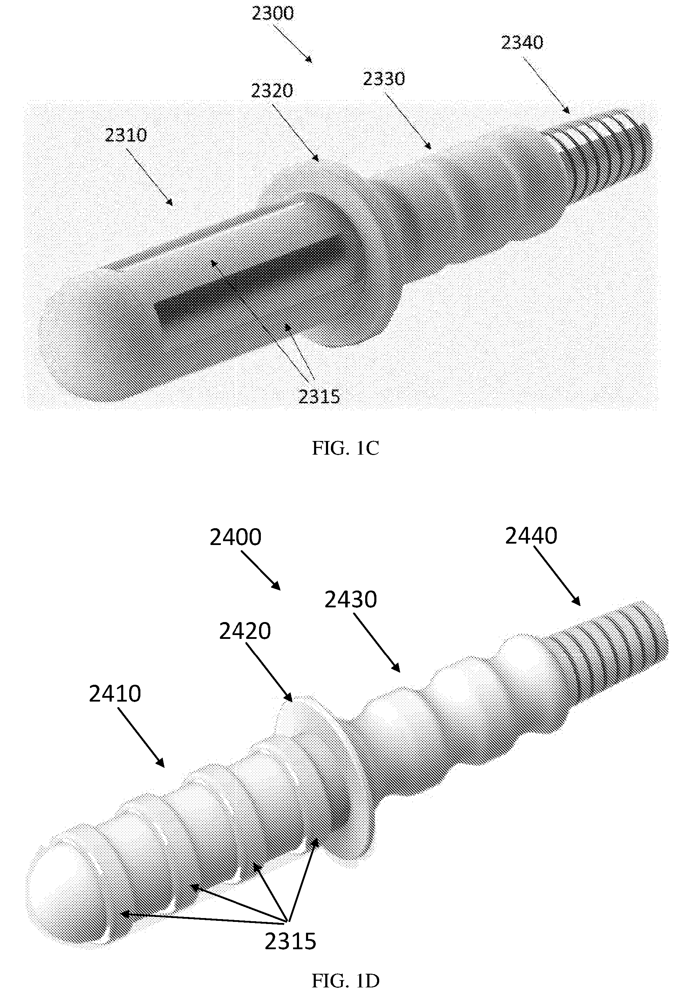

In reference to **Fig. 1C****,** an embodiment of a disposable portion **(2300)** of a device for vaginal use is shown. The disposable portion is the portion for insertion into the vagina; the reusable portion, which comprises a handle mechanism for holding the device during insertion and removal (and possibly during use), the control mechanism for controlling delivery of power to the disposable portion, and, in some embodiments, a mechanism for delivering heating or cooling fluids, is not shown.

The cooling means can be selected from the group consisting of: a Peltier effect cooling device, irrigation with cool water, and means for blowing air across the tissue.

It should be noted that, in some embodiments, the disposable portion is sterilizable and can be reused.

The device **(2300)** comprises a distal section **(2310)** for insertion into the vagina, a ring **(2320)** to limit the depth of penetration, a medial portion **(2330),** and a proximal portion **(2340)** configured to provide connection between the disposable portion **(2300)** and the re-usable portion. The connection provided by the proximal portion **(2340)** includes a mechanical connection and electrical communication; it can also include a fluid connection.

The embodiment of **Fig. 1C** has longitudinal electrodes **(2315).**

The embodiment of **Fig. 1C** has four longitudinal electrodes **(2315);** the number of longitudinal electrodes can be between 1 and about 20, preferably between about 4 and about 10.

In preferred variants, the device comprises sensors to monitor the temperature. This can be the temperature of at least a portion of an electrode, the temperature of the tissue adjacent to a portion of an electrode, and any combination thereof.

In preferred variants of these embodiments, the temperature of the tissue is measured.

In embodiments with at least one temperature sensor, preferably, a sensor will be adjacent to an electrode or inside an electrode so that a temperature measured by a temperature sensor can be associated with the at least a portion of an electrode primarily responsible for inducing the temperature. In preferred variants of embodiments with temperature sensors, there is at least one temperature sensor adjacent to each electrode and the temperature is individually and separately controlled for at least a portion of each electrode. The temperatures of any selected portions of a given pair of electrodes can be the same or they can be different. In preferred variants, the temperatures will be different, with higher temperatures for the distal portions of the electrodes and lower temperatures for the proximal portions of the electrodes. A difference between a device for treating external skin tissue and treating vaginal tissue is that the labial and vulval tissues are much more sensitive to heat than the vaginal tissues. Therefore, it is preferable that the proximal end of the distal section **(2410)** of the device be heated less than the remainder of the distal section **(2410).**

**Fig. 1D** shows an embodiment of a vaginal treatment device **(2400)** in which the electrodes **(2415)** are ring-like, encircling the distal section **(2410)** of the device. The device **of** **Fig. 1D** has a ring **(2420)** to prevent excessive penetration of the device into the vagina, a medial portion **(2430)** and a proximal portion **(2440),** with the proximal portion **(2440)** configured to provide connection between the disposable portion **(2400)** and the re-usable portion (not shown). The connection provided by the proximal portion **(2440)** includes a mechanical connection and electrical communication; it can also include a fluid connection.

The embodiment of **Fig. 1D** has four ring electrodes **(2415);** the number of ring electrodes can be between 1 and about 20, preferably between about 4 and about 10.

In embodiments with at least one temperature sensor, preferably, a sensor will be adjacent to an electrode or inside an electrode so that a temperature measured by a temperature sensor can be associated with the at least a portion of an electrode primarily responsible for inducing the temperature.

Preferably, the temperature sensors are configured to measure the tissue temperature, not the electrode temperature.

In preferred variants of embodiments with temperature sensors, there is at least one temperature sensor adjacent to each electrode and the temperature is individually and separately controlled for at least a portion of each electrode. The temperatures of any selected portions of a given pair of electrodes can be the same or they can be different. In preferred variants, the temperatures will be different, with higher temperatures for central electrodes and lower temperatures for edge electrodes.

In typical embodiments of the present invention, the pulsed electromagnetic frequency is generated by a pulsed electromagnetic frequency generator in communication with a power unit which can be in a handle or in a separate unit. The pulsed electromagnetic frequency generator **(2)** is positioned near the treated tissue and provides electromagnetic pulses to the patient's tissue. In some embodiments, the pulsed electromagnetic frequency generator **(2)** provides an electromagnetic field where the frequency, the RMS average amplitude, the pulse width, and the duty cycle are constant in time. In some embodiments, the pulsed electromagnetic frequency generator **(2)** provides a dynamic electromagnetic field, where at least one of the frequency, the RMS average amplitude, the pulse width, and the duty cycle vary with time.

The pulsed electromagnetic frequency generator is an electromagnetic field generator.

The specific items that are varied in a dynamic magnetic field are chosen according to the specific treatment to be applied. For non-limiting example, to stimulate angiogenesis, pulses at a frequency of about 15 Hz, intensity of about 12 gauss and duration of about 5 ms are generated. In another non-limiting example, to stimulate collagen production, wave pulses at a frequency between about 10 Hz and about 25 Hz and intensity of about 20 gauss are generated.

The tissue diathermy device **(4)** is configured to apply heat to the region of a patient's dermis to heat it to a predetermined temperature T. According to some embodiments of the present invention, the heat is applied by passing electrical current through the tissue. The electrical current can be applied in any combination of the following manners:
1. Through at least one electrode which is in direct physical contact with the tissue;
2. through at least one electrode which is not in physical contact with the tissue, and the electrical current is transferred by induction.
3. through at least one antenna which passes the electrical current to the tissue via electromagnetic induction.

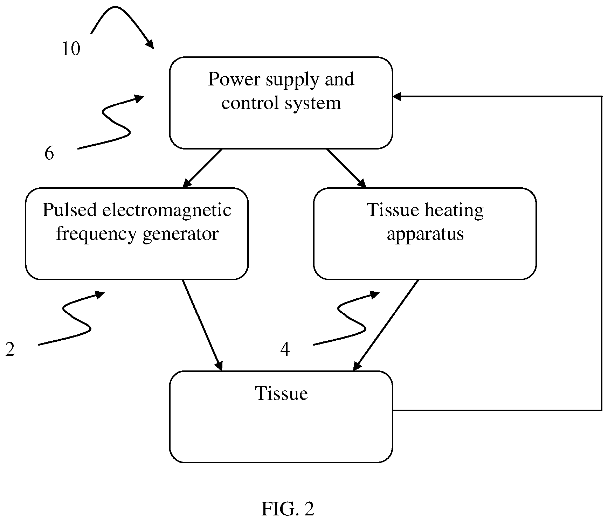

Reference is now made to **Fig. 2****,** which illustrates an embodiment of the present invention, comprising a pulsed electromagnetic frequency generator **(2)** and a tissue diathermy device **(4),** and additionally comprising a control system **(6)** configured to regulate a member of a group consisting of: electromagnetic pulses, tissue diathermy and any combination thereof.

In some embodiments of the present invention, treatment is provided only within safe treatment parameters (Table 1). Use of unsafe treatment parameters (Table 2) is avoided.

Safe treatment parameters are defined by the parameters in table 1:

**Table 1: safe treatment parameters**

| ***parameter*** | **Values** |
|---|---|
| Time, tₒ | 0-600 minutes |
| Temperature, T | 25-80 Celsius |
| Duty cycle tₚ/tₜ | 0-100% |
| PEMF frequency, F | DC - 50 Hz |
| RF frequency, F_{R} | 200 kHz - 10 MHz |
| Power, P | 0-100 W |
| Magnetic field intensity, B | 0-20 Gauss |
| Depth D of treated tissue | ≤30 millimeters |

Unsafe treatment parameters are defined by the parameters in table 2:

**Table 2: unsafe treatment parameters**

| ***parameter*** | **Values** |
|---|---|
| Time, tₒ | >10 hours (nonstop) |
| Temperature, T | >80 Celsius |
| Duty cycle tₚ/tₜ | N/A |
| PEMF frequency, F | >50 Hz |
| RF frequency, F | >10 MHz |
| Power, P | >100 W |
| Magnetic field intensity, B | >20 Gauss |
| Depth D of treated tissue | >30 millimeters |

In some embodiments, the control system **(6)** additionally comprises:
(a) a processor configured to store in a communicable database predetermined parameters defining *(i)* safe treatment parameters and *(ii)* unsafe treatment parameters, with the parameters selected from a group consisting of: total duration of a treatment tₜ, time tₚ during which PEMF is applied, temperature T to which the treated tissue is heated, duty cycle tₚ/tₜ, frequency F, power P applied by the pulses from a pulsed electromagnetic frequency generator, intensity I of RF diathermy, depth D of the treated tissue, magnetic field intensity B, tissue impedance, specific absorption rate (SAR), superficial muscle contractions and any combination thereof;
(b) a sensing mechanism configured to sense electromagnetic radiation and heating parameters selected from a group consisting of: time (duration) tₜ of a treatment, time tₚ during which electromagnetic radiation and/or heating is applied, temperature T to which the treated tissue is heated, duty cycle tₚ/tₜ, frequency F of the applied electromagnetic or heat radiation, power P applied by the pulses from a pulsed electromagnetic frequency generator, intensity I of RF diathermy, depth D of the treated tissue, magnetic field intensity B, tissue impedance, specific absorption rate (SAR), superficial muscle contractions and any combination thereof; and
(c) a regulation mechanism configured to allow pulsed electromagnetic radiation and heat radiation if parameters are within a range of safe treatment parameters and to stop the pulsed electromagnetic radiation if the radiation parameters are outside the range of safe treatment parameters and, therefore, within the range of unsafe treatment parameters.

In some embodiments, the system additionally comprises at least one sensor to monitor at least one physical parameter selected from a group consisting of: total duration of a treatment tₜ, time tₚ during which PEMF is applied, temperature T to which treated tissue is heated, duty cycle tₚ/tₜ, frequency F of the PEMF, power P applied by the pulses of the pulsed electromagnetic frequency generator, depth D of the treated tissue, magnetic field intensity B, tissue impedance, specific absorption rate (SAR), superficial muscle contractions and any combination thereof.

The at least one sensor receives at least one parameter from the treated tissue. From the received parameters, at least one change can be made to at least one operating parameter of the pulsed electromagnetic frequency generator **(2),** at least one operating parameter of the tissue diathermy device **(4),** or both. The change to the at least one operating parameter can be made to optimize the effect of the pulsed electromagnetic field, to optimize the effect of the tissue diathermy, to augment the synergistic effect of both components and any combination thereof, whilst avoiding harm to the tissue.

In some embodiments of the present invention the shape of the electromagnetic pulse is selected in a non-limiting manner from a group consisting of square wave, a sine wave, a triangular wave, a sawtooth wave, a ramp wave, a spiked wave and any combination thereof.

In some embodiments, the system as defined above is configured to provide electromagnetic pulses at a frequency of about 15-16 Hz, with the pulse RMS avaerage intensity varying over time between about 0 and about 12 gauss. In some variants of these embodiments, the pulse RMS average intensity increases over time from about 0 to about 12 gauss, then drops to about 0, whereupon the cycle repeats. In some variants of these embodiments, the RMS average intensity reduces gradually, for non-limiting example, reducing over approximately the same time that it took to increase. In other embodiments, the intensity drops to zero rapidly, over no more than a few pulses and preferably over less than one pulse.

In some embodiments, the system as defined above is configured to provide electromagnetic square wave pulses at a frequency of about 15-16 Hz, with the RMS average magnitude of the pulses repeatedly increasing from about 0 to about 12 gauss.

In some embodiments intended to stimulate collagen production, the system as defined above is configured to provide wave pulses at a frequency between about 10 Hz and about 25 Hz and constant RMS average intensity of about 20 gauss. In some embodiments intended to stimulate collagen production, the wave pulses are triangular. In other embodiments, other waveforms, as disclosed herein, are used.

In some embodiments, the system as defined above is configured to provide alternating current (AC) at a frequency of about 1 MHz.

In some embodiments, the system as defined above is configured to provide intensity of about 80 J/cm²sec.

In some embodiments of the present invention, the duration of each pulse applied by the pulsed electromagnetic frequency generator is in a range between about 3 ms and about 1000 ms.

In some embodiments of the present invention, the frequency F of the pulses of the pulsed electromagnetic frequency generator is in a range between about 1 Hz and about 1 MHz.

In some embodiments of the present invention, the power P applied by the pulses of the pulsed electromagnetic frequency generator is in a range between about 1 W per pulse and about 100 W per pulse.

In some embodiments of the present invention, the tissue diathermy device (4) is selected in a non-limiting manner from a group consisting of electric diathermy, RF diathermy and any combination thereof. In some embodiments of the present invention, the tissue diathermy device (4) is selected in a non-limiting manner from a group consisting of an electromagnetic induction device, a short-wave RF diathermy device, or any other means of controllably heating subcutaneous tissue to a temperature T.

In some embodiments of the present invention, the temperature T to which the tissue is heated is in a range from about 30 degrees C to about 80 degrees C.

In some embodiments of the present invention, the system includes a mechanism for cooling the tissue.

In some embodiments of the present invention, the pulsed electromagnetic frequency generator (2) and the tissue diathermy device (4) have more than one applicator to treat more than one body part simultaneously.

In some embodiments of the present invention, the pulsed electromagnetic frequency generator **(2)** has electrostatic shielding.

Is should be emphasized that the system as defined in any of the embodiments produces synergistic outcomes in the in each of the following time scales: In the short-term (less than about a week), in the intermediate term (about two to three weeks) and in the long term (about a month or longer).

In the short term, contraction and thickening of collagen fibers occurs, which in turn results in an overall tightening of the vaginal tissue.

In the intermediate term, new epidermal cells and new collagen fibers are produced.

In the long term, cellulite is dispersed.

In yet other embodiments of the invention disclosed herein, at least one of the electrodes additionally comprises a hypodermic syringe for penetrating into subcutaneous tissue. By means of such a syringe, a substance with appropriate activity may be injected into the tissue during treatment. Non-limiting examples of such substances include a muscle relaxant, a local anesthetic, etc.

It is also within the scope of the disclosure to disclose a method for providing rejuvenation to the tissue, comprising (a) generating *N* independent signals, where N is either the number of electrodes or the number of pairs of electrodes, of predetermined waveforms, frequencies, amplitudes, and relative phases to control at least one of pulsed electromagnetic frequency generation or tissue diathermy; (b) transmitting each of the *N* independent signals to at least one electrode; (c) placing at least one of the electrodes at least adjacent to the tissue to be treated; and (d) transmitting power carried by the signals to the tissue. In preferred embodiments, the *N* independent signals are phase-shifted relative to one another. The method can performed by using a device according to any of the embodiments described herein, or in any variant of a device described herein.

It is also within the scope of the disclosure wherein the step of generating *N* independent signals further comprises generating *N* independent pulsed electromagnetic fields such that the time-dependent amplitude *A_{t,m}* of the mth of the *N* independent signals is given by the relationship *A_{t,m} = A_{o,m} · Fₘ* (*ωₘt + φₘ*), where *A*_{0,*m*} is a predetermined constant which greater than or equal to 1, *Fₘ* is a predetermined periodic function of time, *ωₘ* is the angular frequency of the *m*th signal, and *φₘ* is a predetermined phase shift of the mth signal. While in preferred embodiments of the method, the step of generating *N* independent signals comprises a step in which *A*₀*_{,m}, Fₘ,* and *ωₘ* are substantially the same for all *N* signals, and *φₘ* is substantially the same for any two pairs of signals *m* and m+1, this restriction is by no means required, and it is within the scope of the disclosure to disclose a method in which any or all of them are not identical for all N signals. In preferred embodiments of the method in which *φₘ* is substantially the same for all pairs of signals *m* and *m+1,* the method further includes a step of providing a phase shift *φₘ* for each of the *N* independent signals according to the relationship φₘ=π*k*(*j*-1)/*N*, where 0 ≤ *k* ≤ 1, *m* = 1, 2, 3...*N* and *j=1, 2, 3..*. ; where *N* is the number of electrodes. For example, for one electrode in a set of four *(N=4), k=1* and the phase is 135 degrees, while, for another electrode in the set of four, the phase will be 45 degrees, and so on.

In some embodiments of the method, it further includes a step of choosing *Fₘ* from the group consisting of sine, cosine, tan, cotangent (cot), sawtooth wave, triangular wave, square wave, rectangular wave, trapezoidal wave, spiked wave, and any combination thereof.

In yet other embodiments of the invention disclosed herein, at least one of the electrodes additionally comprises a hypodermic syringe for penetrating into subcutaneous tissue. By means of such a syringe, a substance with appropriate activity may be injected into the tissue during treatment. Non-limiting examples of such substances include a muscle relaxant, a local anesthetic, etc.

It is also within the scope of the disclosure to disclose a method for providing rejuvenation to the treated tissue, comprising (a) generating *N* independent signals, where N is either the number of electrodes or the number of pairs of electrodes, of predetermined waveforms, frequencies, amplitudes, and relative phases to control at least one of pulsed electromagnetic frequency generation or tissue diathermy; (b) transmitting each of the *N* independent signals to at least one electrode; (c) placing at least one of the electrodes at least adjacent to the tissue to be treated; and (d) transmitting power carried by the signals to the tissue. In preferred embodiments, the *N* independent signals are phase-shifted relative to one another. The method can performed by using a device according to any of the embodiments described herein, or in any variant of a device described herein.

It is also within the scope of the disclosure wherein the step of generating *N* independent signals further comprises generating *N* independent pulsed electromagnetic fields such that the time-dependent amplitude *A_{t,m}* of the mth of the *N* independent signals is given by the relationship *A_{t,m}* = *A*_{0*,m*} · *Fₘ(ωₘt* + *φₘ*), where *A*_{0,*m*} is a predetermined constant which greater than or equal to 1, *Fₘ* is a predetermined periodic function of time, *ωₘ* is the angular frequency of the mth signal, and *φₘ* is a predetermined phase shift of the mth signal. While in preferred embodiments of the method, the step of generating N independent signals comprises a step in which *A*_{0,}*ₘ, Fₘ,* and *ωₘ* are substantially the same for all N signals, and *φₘ* is substantially the same for any two pairs of signals *m* and *m*+1, this restriction is by no means required, and it is within the scope of the disclosure to disclose a method in which any or all of them are not identical for all *N* signals. In preferred embodiments of the method in which *φₘ* is substantially the same for all pairs of signals *m* and *m+1,* the method further includes a step of providing a phase shift *φₘ* for each of the N independent signals according to the relationship φₘ=π*k*(*j*-1)/*N*, where 0 *≤ k ≤* 1, *m* = 1, 2, 3.. *.N* and *j*=*1,2,3... ;;* where *N* is the number of electrodes. For example, for one electrode in a set of four *(N=4), k=1* and the phase is 135 degrees, while, for another electrode in the set of four, the phase will be 45 degrees, and so on.

In preferred embodiments of the method, it further includes a step of choosing *Fₘ* from the group consisting of sine, cosine, tan, cotangent (cot), sawtooth wave, triangular wave, square The heat can be provided to the treated tissue by applying an electrical current through the electrodes which is absorbed by the subcutaneous tissue, or by RF power applied to the tissue.

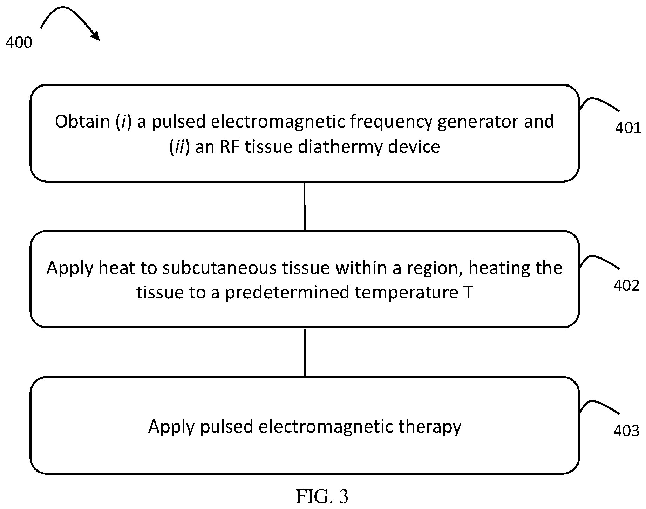

Reference is now made to **Fig. 3****,** schematically illustrating an embodiment of a method **(400)** of increasing rejuvenation of a region of a patient's skin or mucosal tissue. The method comprises steps selected *inter alia* from obtaining (i) a pulsed electromagnetic frequency generator; and, *(ii)* a tissue diathermy device **(401);** applying heat to subcutaneous tissue within the region, heating the region up to a predetermined temperature T **(402);** the temperature T being optimized for producing new collagen and causing dermal proliferation and contracting existing collagen. While the collagen contraction tightens the vaginal tissue immediately, the effects of dermal proliferation and new collagen production will develop later. The next step is applying additional pulsed electromagnetic fields **(403)** which generate a healing mechanism in the heated tissue which includes growth factor production and cytokine release and, eventually, angiogenesis.

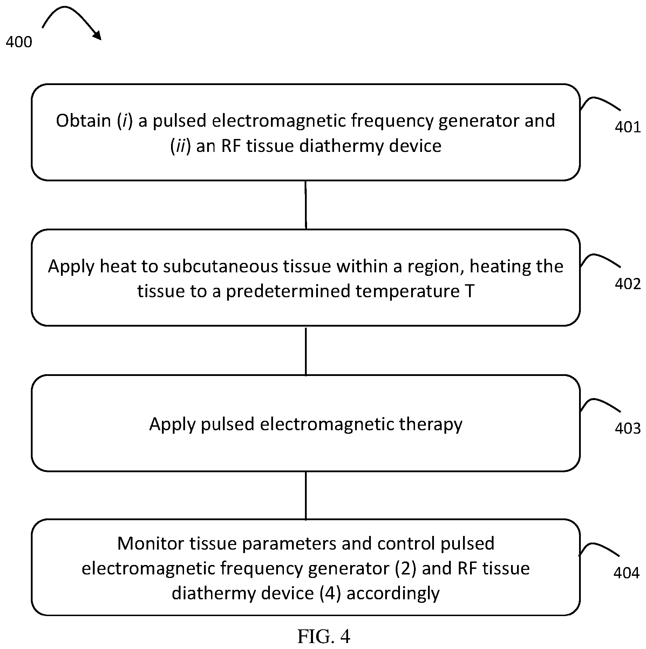

Reference is now made to **Fig. 4****,** which illustrates an embodiment of a preferred method of the present disclosure. According to this embodiment, the method **(400)** additionally comprises a step **(404)** which includes at least one of: monitoring the application of heat to subcutaneous tissue within the region, controlling application of heat to subcutaneous tissue within the region, monitoring application of pulsed electromagnetic therapy to the region and controlling the application of pulsed electromagnetic therapy to the region.

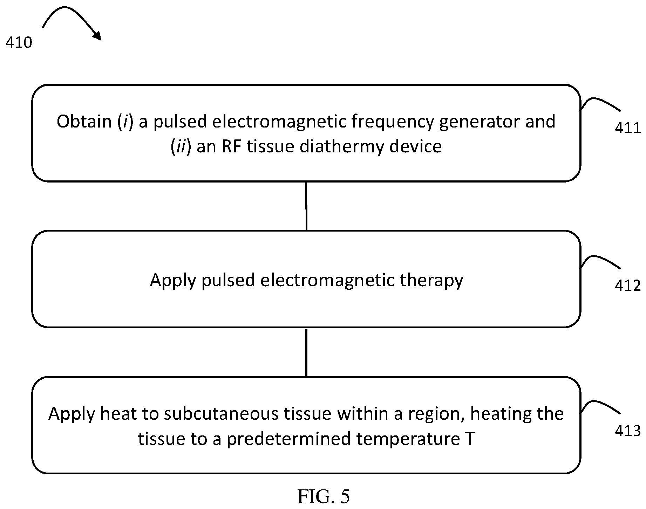

Reference is now made to **Fig. 5****,** schematically illustrating an embodiment of a method **(410)** of increasing tissue rejuvenation in a region of a patient's vaginal tissue. The method comprises steps selected *inter alia* from: obtaining (*i*) a pulsed electromagnetic frequency generator and *(ii)* a tissue diathermy device **(411);** applying a pulsed electromagnetic field **(412)** which generates a healing mechanism in the tissue, which includes release of growth factors and cytokines and eventually angiogenesis; and, finally, applying heat to the subcutaneous tissue within the region, thereby heating the subcutaneous tissue to a temperature T **(413);** the temperature T being optimized for producing new collagen and causing dermal proliferation and contracting collagen. While the collagen contraction tightens the tissue, the effects of dermal proliferation and new collagen production will develop later.

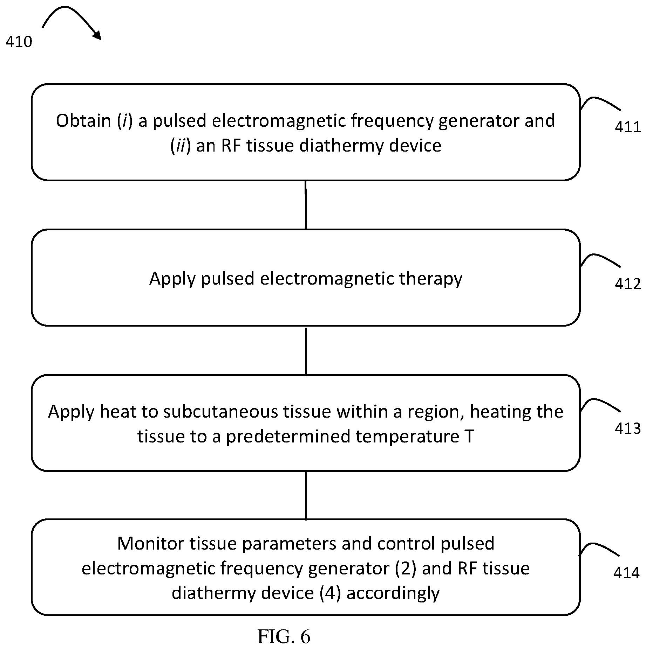

Reference is now made to Fig. 6, which illustrates another embodiment of a method of the present disclosure. According to this embodiment, the method **(410)** additionally comprises a step **(414)** which includes at least one of: monitoring the application of heat to subcutaneous tissue within the region, controlling application of heat to subcutaneous tissue within the region, monitoring application of pulsed electromagnetic therapy to the region and controlling the application of pulsed electromagnetic therapy to the region.

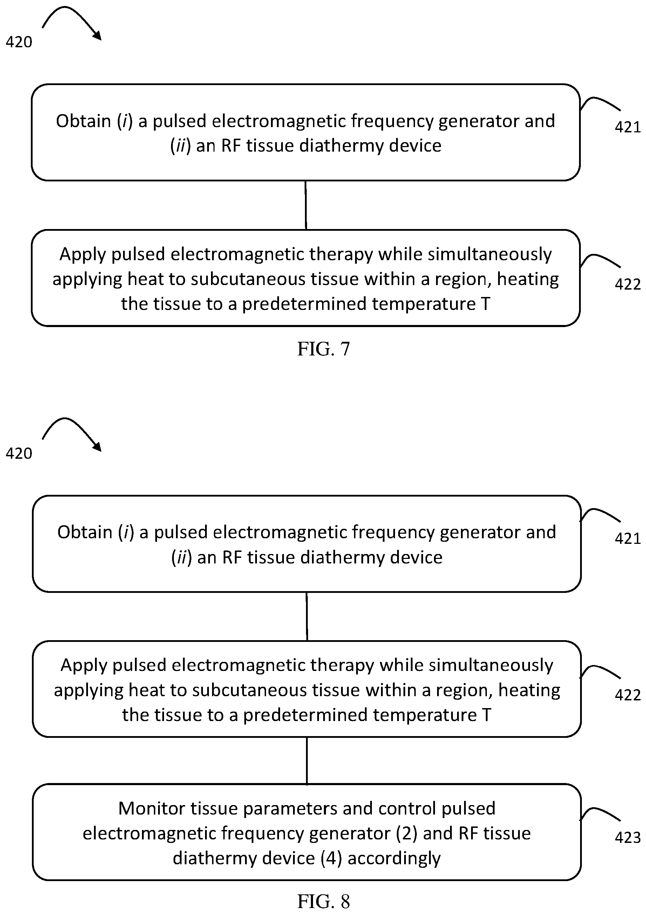

Reference is now made to **Fig. 7****,** schematically illustrating an embodiment of a method **(420)** of increasing rejuvenation of a region of skin or mucosal tissue. The method comprises steps selected *inter alia* from: obtaining (i) a pulsed electromagnetic frequency generator and, *(ii)* a tissue diathermy device **(421);** and applying a pulsed electromagnetic field **(422)** whilst simultaneously applying heat to subcutaneous tissue within the region, heating the tissue to a temperature T. The electromagnetic pulses generate a healing mechanism in the heated tissue, which includes growth factor and cytokine release and eventually angiogenesis. The temperature T is optimized for producing new collagen and causing dermal proliferation and contracting collagen. While the collagen contraction tightens the affected tissue immediately, the effects of dermal proliferation and new collagen production will develop later.

Reference is now made to **Fig. 8****,** which illustrates a preferred embodiment of a method of the present disclosure. According to this embodiment, the method **(420)** additionally comprises a step **(424)** which includes at least one of: monitoring the application of heat to subcutaneous tissue within the region, controlling application of heat to subcutaneous tissue within the region, monitoring application of pulsed electromagnetic therapy to the region and controlling the application of pulsed electromagnetic therapy to the region.

In some embodiments of the present disclosure, each of the methods as defined above additionally comprises a step of selecting the temperature T to be in a range of about 30 degrees C to about 80 degrees C.

In some embodiments of the present disclosure, each of the methods as defined above additionally comprises a step of applying a dynamic electromagnetic field in said region, where the peak intensity of the electromagnetic field varies with time.

In some embodiments of the present disclosure, each of the methods as defined above additionally comprises steps of:
a. storing in a communicable database predetermined parameters defining (i) safe treatment parameters and *(ii)* unsafe treatment parameters; the parameters are selected from a group consisting of: total duration of a treatment tₜ, time tₚ during which PEMF is applied, temperature T of the treated tissue, duty cycle tₚ/tₜ, frequency F, power P applied by the pulses from a pulsed electromagnetic frequency generator, depth D of said treated tissue, magnetic field intensity B, tissue impedance, specific absorption rate (SAR), superficial muscle contractions and any combination thereof;
b. sensing electromagnetic radiation and heating parameters selected from a group consisting of: total duration of a treatment tₜ, time tₚ during which PEMF is applied, temperature T of the treated tissue, duty cycle tₚ/tₜ, frequency F of the applied radiation, power P applied by the pulses from a pulsed electromagnetic frequency generator, intensity I of the heating, depth D of treated tissue, magnetic field intensity B, tissue impedance, specific absorption rate (SAR), superficial muscle contractions and any combination thereof;
c. allowing the electromagnetic radiation and the heating if the parameters are within said safe treatment parameters and stopping the electromagnetic radiation and the heating if the parameters are in the unsafe treatment parameters.

In some embodiments of the present disclosure, the step of applying heat is performed by a device selected from a group consisting of: an electromagnetic induction device, a device configured to apply direct heat, or from any other means of heating subcutaneous tissue to a temperature T.

In some embodiments of the present disclosure, each of the methods as defined above additionally comprises a step of selecting the frequency F of the pulses applied during the step of applying pulsed electromagnetic therapy to the region to be in a range from about 1 Hz to about 1 MHz.

In some embodiments of the present disclosure, each of the methods as defined above additionally comprises a step of selecting the power P applied during the step of applying pulsed electromagnetic therapy to the region to be in a range from about 1 W per pulse to about 100 W per pulse.

In some embodiments of the present disclosure, in each of the methods as defined above the step of applying heat lasts in a range from about 0.01 minutes to about 100 minutes.

In some embodiments of the present disclosure, in each of the methods as defined above, a pulsed electromagnetic field is applied for a time in a range from about 0.01 minutes to about 100 minutes.

In some embodiments of the present disclosure, in each of the methods as defined above, the steps of applying heat and applying the pulsed electromagnetic therapy are carried out in a manner selected from a group consisting of: simultaneously, sequentially (alternating applying heat and applying pulsed electromagnetic therapy) or separately (applying heat in one phase of a treatment and pulsed electromagnetic therapy in a separate phase of the treatment)

In some embodiments of the present disclosure, in each of the methods as defined above, the method is repeated 1 to 100 times in each treatment.

In some embodiments of the present disclosure, a typical protocol for the pulsed electromagnetic frequency generator **(4)** includes, for non-limiting example, a preset number of about 1 µs pulses with a duty cycle of about 50% and a pause of up to about 250 µs. In protocols of this type, power is supplied to the tissue during the period in which the preset number of pulses is applied.

In some embodiments of the present disclosure, a typical protocol for the pulsed electromagnetic frequency generator **(4)** includes, for non-limiting example, about 10 pulses of about 1 µs period with about 50% duty cycle and a preset pause of up to about 512 µs. In protocols of this type, power is supplied to the tissue during the period in which the preset number of pulses is applied.

In some embodiments of the present disclosure, a typical protocol for the pulsed electromagnetic frequency generator **(4)** includes, for non-limiting example, a variant of the previous protocol, wherein the number of pulses administered is a multiple of about 10.

In some embodiments of the present disclosure, in each of the methods as defined above, the treatment is repeated more than once.

In some embodiments of the present disclosure, each of the methods as defined above additionally comprises a step of selecting the shape of the electromagnetic pulse from a group consisting of: a square wave, a sine wave, a triangular wave, a sawtooth wave, a ramp wave, a spiked wave and any combination thereof.

It should also be appreciated that the above described description of methods and apparatus are to be interpreted as including apparatus for carrying out the methods, and methods of using the apparatus of any type as are known to a person or ordinary skill in the art, and which need not be described in detail herein for enabling a person of ordinary skill to practice the invention.

For the main embodiments of the invention, the particular selection of type and model is not critical, although, where specifically identified, this may be relevant. The present invention has been described using detailed descriptions of embodiments thereof that are provided by way of example and are not intended to limit the scope of the invention. No limitation, in general, or by way of words such as "may", "should", "preferably", "must", or other term denoting a degree of importance or motivation, should be considered as a limitation on the scope of the claims unless expressly present in such claim as a literal limitation on its scope. It should be understood that features and steps described with respect to one embodiment can be used with other embodiments and that not all embodiments of the invention have all of the features and/or steps shown in a particular figure or described with respect to one of the embodiments. That is, the disclosure should be considered complete from combinatorial point of view, with each embodiment of each element considered disclosed in conjunction with each other embodiment of each element (and indeed in various combinations of compatible implementations of variations in the same element). Variations of embodiments described will occur to persons of the art. Furthermore, the terms "comprise," "include," "have" and their conjugates, shall mean, when used in the claims, "including but not necessarily limited to." Each element present in the claims in the singular shall mean one or more element as claimed, and when an option is provided for one or more of a group, it shall be interpreted to mean that the claim requires only one member selected from the various options, and shall not require one of each option.

It is noted that some of the above described embodiments describe the best mode contemplated by the inventors and therefore can include structure, acts or details of structures and acts that may not be essential to the invention and which are described as examples. Structure and acts described herein are replaceable by equivalents performing the same function, even if the structure or acts are different, as is known in the art. Therefore, the scope of the invention is limited only by the elements and limitations as used in the claims.

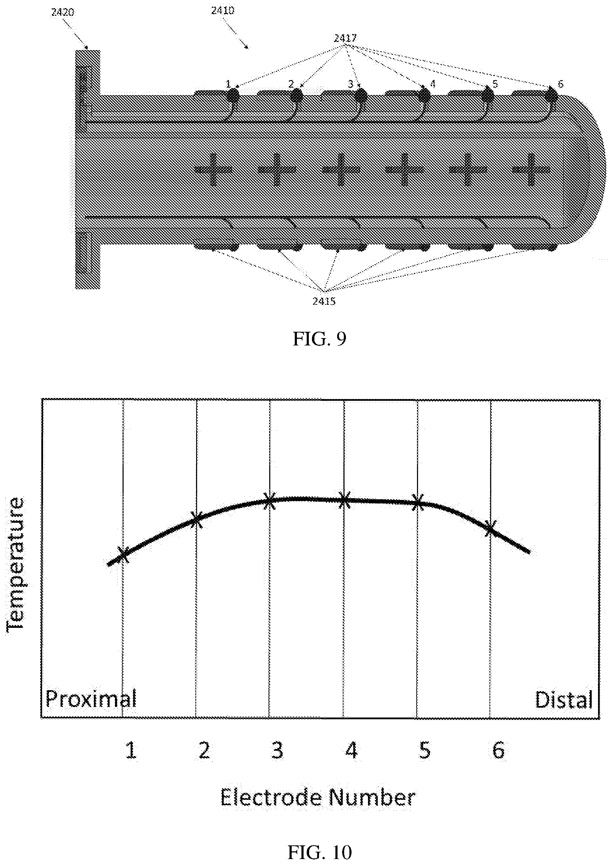

Fig. 9 shows a cross-section of a device with 6 ring electrodes (2415) encircling the distal section of the device (2410); the ring (2420) to prevent excessive penetration of the device into the vagina is also shown, A temperature sensor (2417) is shown adjacent to each ring electrode (2415).

In preferred embodiments of devices with ring electrodes, a predefined temperature profile can be created within the vagina. Typically, at least one predefined temperature profile can be stored in a database in communication with a processor, with the processor in communication with at least one temperature sensor and at least one power supply; the power supply providing power to the electrodes.

In preferred embodiments, both PEMF and heating are supplied to the electrodes, with the heating mechanism selected from a group consisting of tissue diathermy, electric current, an inductive electromagnetic field and any combination thereof. In preferred embodiments, the heating mechanism is RF tissue diathermy.

The temperature can vary longitudinally along the distal section (2410) of the device, radially around the distal section (2410) of the device and any combination thereof. Typically, the temperature profile is created via feedback, with a comparison between the temperature measured by each sensor and a predefined, stored temperature profile being used to adjust the power applied to each electrode.

Fig. 10 shows an exemplary temperature profile, where the temperature varies longitudinally along the distal section (2410) of the device. The numbers 1 to 6 refer to the 6 electrodes shown in Fig. 9, with 1 being the most proximal electrodes and 6 the most distal electrode. The temperature is lowest at electrode 1, which is closest to the heat-sensitive vulva and labia. In this example, the temperature of the distal-most electrode, electrode 6, is also lower than that of the central electrodes,

Under feedback control, more RF power will be supplied to electrodes 3, 4 and 5 and less power will be supplied to electrodes 1, 2 and 6 so as to maintain the desired temperature profile in the vagina.

In some embodiments, the temperature in the vagina will be between about 30 degrees C and about 80 degrees C during treatment, preferably between about 40 degrees C and about 50 degrees C.

As can be seen from Figs. 1B, 1C, 1D and 9, the electrodes and sensors can be enclosed in a platform, to maintain them in a desired position. Similarly, the processor and regulating means can be enclosed in a platform. The processor/regulating mechanism platform can be the same as the electrode/sensor platform, reversibly connected to it, or permanently connected to it. Either platform can additionally comprise a power supply.

In some embodiments, the device can provide at least one of fractional PEMF and fractional tissue diathermy, with only a fraction of the electrodes activated at any one time. This can reduce discomfort to the patient during treatment.

In some embodiments, the device can additionally comprise a mechanism to induce suction, so as to induce at least a portion of the tissue to come into intimate contact with the device. In some embodiments, the device will comprise at least one port. When the device is in use, suction is applied to at least one of the ports, causing the tissue to be drawn into intimate contact with the device and, if a port opens into a recess in the device, causing the tissue to be drawn into the recess in the device. In preferred embodiments, the tissue is drawn in to a sufficient extent that it makes physical contact with the ports and any electrodes or transducers disposed thereabout.

The suction unit is configured to operate either continuously or in pulses, delivering either a constant suction, or a pulsed suction to the tissue.

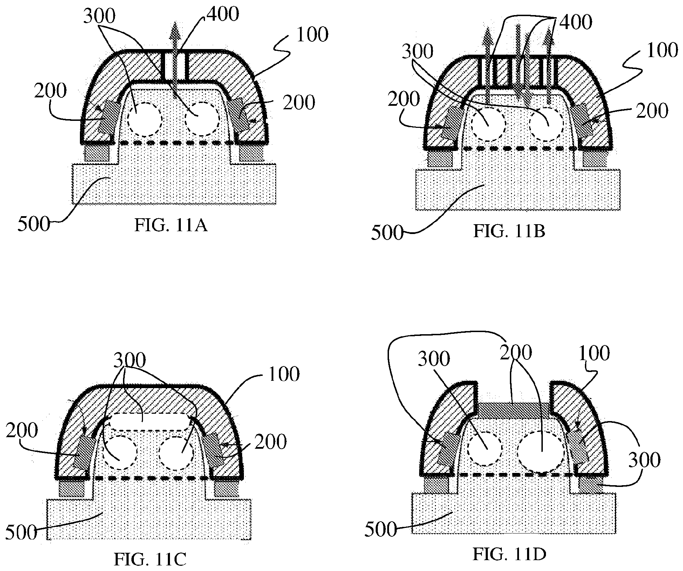

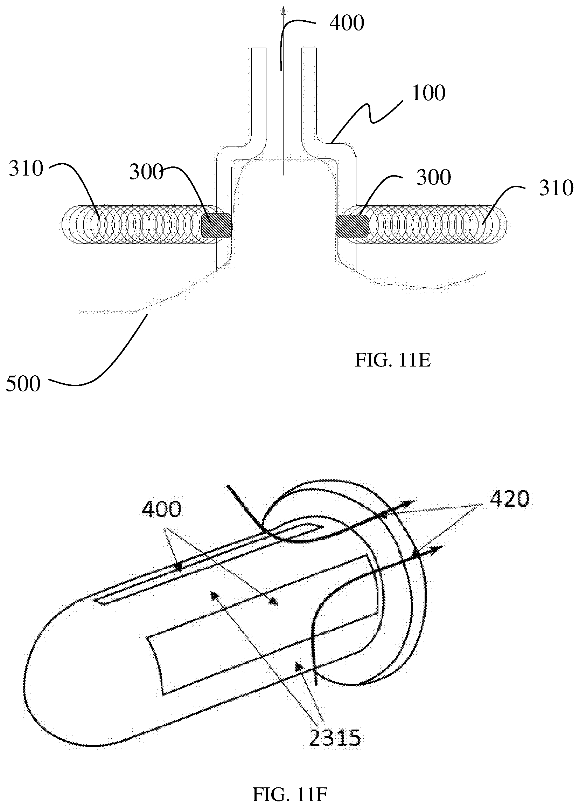

Fig. 11 provides schematic cross-sectional views of five exemplary embodiments of the device that provide non-limiting illustrations of possible geometries of the device. While these illustrations only show the elements that are disposed in the plane of the cross-section and a hidden view of a limited number of elements hidden by tissue 500 that has been drawn into the housing, it is emphasized that the only limitations on the number of tissue diathermy devices and pulsed electromagnetic frequency generators that can be used are the physical size of the housing and the minimum distance necessary to prevent short-circuiting. In the embodiments shown, the tissue diathermy devices and pulsed electromagnetic frequency generators are shown as separate; in some embodiments, the same electrodes produce both tissue diathermy device and pulsed electromagnetic fields.

Fig. 11A shows an embodiment in which there is a single port 400; the arrow indicates the direction of air flow when the device is in fluid connection with a source of vacuum. Tissue diathermy devices 200 are disposed on the lip and interior surface of the housing, while pulsed electromagnetic frequency generators 300 are disposed on the interior surface of the housing. **Fig. 11B** illustrates an embodiment in which the tissue diathermy devices and transducers are disposed as shown in **Fig. 11A****,** but in which a plurality of ports **400** are provided. The arrows indicate the direction of air flow when the device is in fluid connection with a source of vacuum and when the air is let back in after the treatment has concluded.

**Fig. 11C** shows an embodiment in which at least one transducer is disposed about the substantially closed end of the housing. **Fig. 11D** shows an embodiment in which both pulsed electromagnetic frequency generators and tissue diathermy devices are disposed about the lip of the housing and both pulsed electromagnetic frequency generators and tissue diathermy devices are disposed about its inner surface. In this embodiment, at least one tissue diathermy device is disposed about the substantially closed end of the housing. In the embodiments illustrated in **Figs. 11C** and **11D****,** the ports displaced from the central axis of the housing, and hence are not shown in the figures.

**Fig. 11E** presents a schematic illustration of an embodiment of the invention. In this embodiment, the housing has a generally cylindrical rather than cup-shaped housing, and the pulsed electromagnetic frequency generators are disposed about the interior surfaces of the sides of the housing, as shown in the figure.

**Fig. 11F** presents a schematic illustration of the distal end of an embodiment of the invention with a cylindrical housing, with longitudinal electrodes **(2315)** on the exterior of the housing and ports **(400)** between the electrodes **(2315).** Airflow to cause suction is schematically illustrated by the arrows **(420).**

In some embodiments, the device additionally comprises a recess configured to be placed against or in proximity to the surface of the skin or mucosal tissue.

In the some embodiments, the tissue is drawn into the recess to a depth sufficient that physical contact is made with any tissue diathermy applicators or pulsed electromagnetic frequency electrodes disposed about the inner surface of the recess, and with the ports themselves.

In the some embodiments, the tissue is drawn into the recess without being in physical contact with any tissue diathermy applicators or pulsed electromagnetic frequency electrodes disposed about the inner surface of the housing.

## Claims

1. An integrated system (10, 20) for increasing rejuvenation of at least one region of a patient, said at least one region selected from a group consisting of said patient's skin, said patient's mucosal tissue and any combination thereof, said system comprising:
at least two electrodes (41, 2315, 2415) configured to be placed on at least one said region of said patient;
an electromagnetic field generator (2) configured to generate electromagnetic field pulses;
a radiofrequency, RF, generator configured to generate RF power;
at least one temperature sensor, said at least one temperature sensor configured to measure at least one temperature in at least one portion of said at least one region of said patient;
a database configured to store at least one temperature profile comprising at least one temperature and at least one predetermined region of said patient;
a processor in communication with said database and said at least one temperature sensor, and
a control system (6);
wherein:
none of said at least two electrodes is configured to penetrate said at least one region;
each of said at least two electrodes (41, 2315, 2415) is configured to provide electromagnetic pulses and RF power to said at least one region, said RF generator configured to apply heat to said at least one region;
said control system (6) is configured to control at least one of said electromagnetic field generator (2) or application of said heat by said electrodes (41, 2315, 2415);
said control system (6) is configured to apply said electromagnetic pulses to said at least one region and apply said heat to said at least one region in a manner selected from a group consisting of: simultaneously, sequentially, separately and any combination thereof;
wherein said processor is configured to feedback control RF output to said pulsed electromagnetic frequency generator (2) and said RF generator to maintain said temperature profile in said at least one region; and,
wherein applying said electromagnetic pulses and said heat to said at least one region is carried out such that the control system (6) maintains at least a first portion of said at least one region of mucosal tissue within a predetermined temperature range T₁ and maintains at least a second portion of said at least one region of said mucosal tissue within a predetermined temperature range T₂.

2. The system of claim 1, wherein at least one of the following is held true:
a. said at least one region of said patient is within a vagina;
b. wherein at least one temperature in said temperature profile is within said range of about 30 degrees to about 80 degrees C;
c. said system is configured to provide a dynamic magnetic field such that a magnitude of said electromagnetic pulses are variable with time;
d. said control system (6) is configured to monitor physical tissue parameters and to change a member of a group consisting of: said applied heat, said electromagnetic pulses and any combination thereof accordingly;
e. a duration of each pulse applied by said system ranges between about 3 ms and about 1000 ms;
f. said RF energy is applied in the form of RF pulses, wherein a frequency F of the RF pulses of said system ranges between about 200 kHz and about 1MHz;
g. power P applied by the pulses of said system ranges between about 1 W and about 100 W of RMS average power; and
h. said predetermined temperature range is from about 40 degrees C to about 50 degrees C.

3. The system of claim 2, wherein said at least one temperature sensor is selected from a group consisting of: a thermistor, a thermocouple and any combination thereof.

4. The system of claim 3, wherein said RF generator comprises a RF tissue diathermy device.

5. The system of claim 1, wherein:
a frequency F of said pulses from said pulsed electromagnetic field generator is in a range between about 1 Hz and about 50 Hz.

6. The system of claim 1, wherein:
pulsed electromagnetic power from said pulsed electromagnetic field generator (2) comprises pulses at a frequency F of about 15 Hz, and a magnetic field intensity B of about 12 gauss.

7. The system of claim 1, wherein at least one of the following is being held true:
a. said control system (6) includes a cooling mechanism for cooling said region of mucosal tissue; and
b. said system comprises at least one platform, said at least two electrodes being encased in said at least one platform.

8. The system of claim 1, wherein all said electrodes (41, 2315, 2415) are configured to simultaneously apply said RF power to said region of mucosal tissue.

9. The system of claim 1, wherein said control system is configured to control said at least two electrodes are independently controlled.

10. The system of claim 1, wherein T₁ is different than T₂.

11. The system of claim 1, wherein the at least one temperature senor is at least two temperature sensors and one of said at least two temperature sensors is positioned adjacent to each of the at least two electrodes.

## Patentansprüche

1. Ein integriertes System (10, 20) zur Steigerung der Verjüngung mindestens eines Bereichs eines Patienten, wobei der mindestens eine Bereich aus einer Gruppe ausgewählt ist, die aus der Haut des Patienten, dem Schleimhautgewebe des Patienten und einer beliebigen Kombination davon besteht, wobei das System umfasst: mindestens zwei Elektroden (41, 2315, 2415), die zum Platzieren auf mindestens einem der genannten Bereiche des Patienten ausgebildet sind;
einen Generator für elektromagnetische Felder (2), der zum Erzeugen elektromagnetischer Feldimpulse ausgebildet ist;
einen Radiofrequenz-(RF-)Generator, der zum Erzeugen von RF-Leistung ausgebildet ist;
mindestens einen Temperatursensor, wobei der mindestens eine Temperatursensor konfiguriert ist zum Messen von mindestens einer Temperatur in mindestens einem Abschnitt des mindestens einen Bereichs des Patienten;
eine Datenbank, die konfiguriert ist zum Speichern von mindestens einem Temperaturprofil, das mindestens eine Temperatur und mindestens einen vorgegebenen Bereich des genannten Patienten umfasst;
einen Prozessor, der mit der genannten Datenbank und dem genannten mindestens einen Temperatursensor in Verbindung steht, und
ein Steuerungssystem (6);
wobei:
keine der mindestens zwei Elektroden zum Eindringen in den mindestens einen Bereich ausgebildet ist;
jede der mindestens zwei Elektroden (41, 2315, 2415) zum Zuführen elektromagnetischer Impulse und RF-Leistung zu dem mindestens einen Bereich ausgebildet ist,
wobei der RF-Generator zum Zuführen von Wärme zu dem mindestens einen Bereich ausgebildet ist;
das Steuerungssystem (6) konfiguriert ist zum Steuern mindestens eines von Folgendem: des Generators für elektromagnetische Felder (2) oder der Zufuhr der Wärme durch die Elektroden (41, 2315, 2415);
das genannte Steuerungssystem (6) konfiguriert ist zum Anwenden der genannten elektromagnetischen Impulse auf den genannten mindestens einen Bereich und zum Zuführen der genannten Wärme zu dem genannten mindestens einen Bereich in einer Weise, die aus einer Gruppe ausgewählt ist, die aus gleichzeitiger, aufeinanderfolgender, getrennter Anwendung und einer beliebigen Kombination davon besteht;
wobei der Prozessor konfiguriert ist zum Durchführen einer Rückkopplungsregelung der RF-Ausgangsleistung an den Generator für gepulste elektromagnetische Frequenzen (2) und an den RF-Generator, um das Temperaturprofil in dem mindestens einen Bereich aufrechtzuerhalten; und,
wobei das Anlegen der elektromagnetischen Impulse und der Wärme an den mindestens einen Bereich derart erfolgt, dass das Steuerungssystem (6) mindestens einen ersten Abschnitt des mindestens einen Bereichs des Schleimhautgewebes innerhalb eines vorgegebenen Temperaturbereichs T₁ und mindestens einen zweiten Abschnitt des mindestens einen Bereichs des Schleimhautgewebes innerhalb eines vorgegebenen Temperaturbereichs T₂ hält.

2. System nach Anspruch 1, wobei mindestens eines von Folgendem zutrifft:
a. dass sich der mindestens eine Bereich des Patienten in einer Vagina befindet;
b. wobei mindestens eine Temperatur in dem genannten Temperaturprofil innerhalb des genannten Bereichs von etwa 30 Grad bis etwa 80 Grad Celsius liegt;
c. das genannte System zum Erzeugen eines dynamischen Magnetfelds ausgebildet ist, sodass der Betrag der elektromagnetischen Impulse zeitlich variabel ist;
d. das genannte Steuerungssystem (6) konfiguriert ist zum Überwachen physikalischer Gewebeparameter und zum entsprechenden Verändern eines Elements aus einer Gruppe, die aus der zugeführten Wärme, den elektromagnetischen Impulsen und einer beliebigen Kombination davon besteht;
e. Die Dauer jedes von dem genannten System angelegten Impulses liegt zwischen etwa 3 ms und etwa 1000 ms;
f. wobei die genannte RF-Energie in Form von RF-Impulsen angelegt wird, wobei die Frequenz F der RF-Impulse des Systems zwischen etwa 200 kHz und etwa 1 MHz liegt;
g. Die von den Impulsen des genannten Systems angelegte Leistung P liegt im Bereich zwischen etwa 1 W und etwa 100 W mittlerer RMS-Leistung; und
h. Der genannte vorgegebene Temperaturbereich liegt zwischen etwa 40 Grad Celsius und etwa 50 Grad Celsius.

3. System nach Anspruch 2, wobei der mindestens eine Temperatursensor aus einer Gruppe ausgewählt ist, die aus einem Thermistor, einem Thermoelement und einer beliebigen Kombination davon besteht.

4. System nach Anspruch 3, wobei der RF-Generator eine RF-Gewebediathermievorrichtung umfasst.

5. System nach Anspruch 1, wobei:
die Frequenz F der Impulse des Generators für gepulste elektromagnetische Felder in einem Bereich zwischen etwa 1 Hz und etwa 50 Hz liegt.

6. System nach Anspruch 1, wobei:
die gepulste elektromagnetische Leistung des Generators für gepulste elektromagnetische Felder (2) Impulse mit einer Frequenz F von etwa 15 Hz und einer Magnetfeldstärke B von etwa 12 Gauss umfasst.

7. System nach Anspruch 1, wobei mindestens eines von Folgendem zutrifft:
a. das genannte Steuerungssystem (6) einen Kühlmechanismus zum Kühlen des Bereichs des Schleimhautgewebes einschließt; und
b. das genannte System mindestens eine Plattform umfasst, wobei die genannten mindestens zwei Elektroden in der mindestens einen genannten Plattform untergebracht sind.

8. System nach Anspruch 1, wobei alle Elektroden (41, 2315, 2415) zum gleichzeitigen Anlegen der RF-Leistung an den Bereich des Schleimhautgewebes ausgebildet sind.

9. System nach Anspruch 1, wobei das Steuerungssystem konfiguriert ist zum unabhängigen Steuern der mindestens zwei Elektroden.

10. System nach Anspruch 1, wobei T₁ sich von T₂ unterscheidet.

11. System nach Anspruch 1, wobei es sich bei dem mindestens einen Temperatursensor um mindestens zwei Temperatursensoren handelt und einer der mindestens zwei Temperatursensoren benachbart zu jeder der mindestens zwei Elektroden angeordnet ist.

## Revendications

1. Système intégré (10, 20) destiné à augmenter le rajeunissement d'au moins une région d'un patient, ladite au moins une région étant sélectionnée à partir d'un groupe constitué de la peau dudit patient, du tissu muqueux dudit patient et d'une quelconque combinaison de ceux-ci, ledit système comprenant : au moins deux électrodes (41, 2315, 2415) configurées pour être placées sur au moins une desdites régions dudit patient ;
un générateur de champ électromagnétique (2) configuré pour générer des impulsions de champ électromagnétique ;
un générateur de radiofréquence, RF, configuré pour générer une puissance RF ;
au moins un capteur de température, ledit au moins un capteur de température étant configuré pour mesurer au moins une température dans au moins une partie de ladite au moins une région dudit patient ;
une base de données configurée pour stocker au moins un profil de températures comprenant au moins une température et au moins une région prédéterminée dudit patient ;
un processeur en communication avec ladite base de données et ledit au moins un capteur de température, et
un système de commande (6) ;
dans lequel :
aucune desdites au moins deux électrodes n'est configurée pour pénétrer dans ladite au moins une région ;
chacune desdites au moins deux électrodes (41, 2315, 2415) est configuée pour fournir des impulsions électromagnétiques et une puissance RF à ladite
au moins une région, ledit générateur RF étant configuré pour appliquer de la chaleur à ladite au moins une région ;
ledit système de commande (6) est configuré pour commander au moins un dudit générateur de champ électromagnéitique (2) ou une application de ladite chaleur par lesdites électrodes (41, 2315, 2415) ;
ledit système de commande (6) est configuré pour appliquer lesdites impulsions électromagnétiques à ladite au moins une région et appliquer ladite chaleur à ladite au moins une région d'une manière sélectionnée à partir d'un groupe constitué de : simultanément, séquentiellement, séparément et d'une quelconque combinaison de ceux-ci ;
dans lequel ledit processeur est configuré pour renvoyer une sortie RF de commande audit générateur de fréquences électromagnétiques pulsées (2) et audit générateur RF pour maintenir ledit profil de températures dans ladite au moins une tégion ; et,
dans lequel l'application desdites impulsions électromagnétiques et de ladite chaleur à ladite au moins une région est réalisée de telle sorte que le système de commande (6) maintient au moins une première partie de ladite au moins une région de tissu muqueux au sein d'une plage de températures prédéterminées T₁ et maintient au moins une seconde partie de ladite au moins une région dudit tissu muqueux au sein d'une plage de températures prédéterminées T₂.

2. Système selon la revendication 1, dans lequel au moins une des conditions suivantes est vérifiée :
a. ladite au moins une région dudit patient se situe au sein d'un vagin ;
b. dans lequel au moins une température dans ledit profil de températures se situe au sein de ladite plage d'environ 30 degrés à environ 80 degrés C ;
c. ledit système est configuré pour fournir un champ magnétique dynamique de telle sorte qu'une amplitude desdites impulsions électromagnétiques est variable dans le temps ;
d. ledit système de commande (6) est configuré pour surveiller des paramètres de tissus physiques et pour changer un membre d'un groupe constitué : de ladite chaleur appliquée, desdites impulsions électromagnétiques et d'une quelconque combinaison de celles-ci en conséquence ;
e. une durée de chaque imppulsion appliquée par ledit système s'échelonne entre environ 3 ms et environ 1 000 ms ;
f. ladite énergie RF est appliquée sous la forme d'impulsions RF, dans lequel une fréquence F des impulsions RF dudit système s'échelonne entre environ 200 KHz et environ 1 MHz ;
g. une puissance P appliquée par les impulsions dudit système s'échelonne entre environ 1 W et environ 100 W de puissance moyenne RMS ; et
h. ladite plage de températures prédéterminées s'échelonne d'environ 40 degrés C à environ 50 degrés C.

3. Système selon la revendication 2, dans lequel ledit au moins un capteur de température est sélectionné à partir d'un groupe constitué : d'une thermistance, d'un thermocouple et d'une quelconque combinaison de ceux-ci.

4. Système selon la revendication 3, dans lequel ledit générateur RF comprend un dispositif de diathermie tissulaire par radiofréquence.

5. Système selon la revendication 1, dans lequel :
une fréquence F desdites impulsions provenant dudit générateur de champ électromagnétique pulsé se situe dans une plage entre environ 1 Hz et environ 50 Hz.

6. Système selon la revendication 1, dans lequel :
une puissance électromagnétique pulsée en provenance dudit générateur de champ électromagnétique pulsé (2) comprend des impulsions à une fréquence F d'environ 15 Hz et une intensité de champ magnétique B d'environ 12 gauss.

7. Système selon la revendication 1, dans lequel au moins une des conditions suivantes est vérifiée :
a. ledit système de commande (6) inclut un mécanisme de refroidissement destiné à refroidir ladite région de tissu muqueux ; et
b. ledit système comprend au moins une plate-forme, lesdites au moins deux électrodes étant logées dans ladite au moins une plate-forme.

8. Système selon la revendication 1, dans lequel toutes lesdites électrodes (41, 2315, 2415) sont configurées pour appliquer simultanément ladite puissance RF à ladite région de tissu muqueux.

9. Système selon la revendication 1, dans lequel ledit système de commande est configuré pour commander lesdites au moins deux électrodes de manière indépendante.

10. Système selon la revendication 1, dans lequel T₁ est différent de T₂.

11. Système selon la revendication 1, dans lequel l'au moins un capteur de température est au moins deux capteurs de températures et un desdits au moins deux capteurs de températures est positionné de manière adjacente à chacune des au moins deux électrodes.
